(19) Europäisches Patentamt
European Patent Office
Office européen des brevets

(11) Numéro de publication: **0 405 427 B1**

# (12) FASCICULE DE BREVET EUROPEEN

(45) Date de publication de fascicule du brevet: **29.03.95**

(51) Int. Cl.[6]: **C07C 47/546**, C07C 49/792, A61K 7/46, C11B 9/00

(21) Numéro de dépôt: **90112089.9**

(22) Date de dépôt: **26.06.90**

(54) **Nouveaux composés aromatiques, leur procédé de préparation et leur utilisation à titre d'ingrédients parfumants.**

(30) Priorité: **30.06.89 CH 2454/89**

(43) Date de publication de la demande:
**02.01.91 Bulletin 91/01**

(45) Mention de la délivrance du brevet:
**29.03.95 Bulletin 95/13**

(84) Etats contractants désignés:
**CH DE FR GB LI NL**

(56) Documents cités:
**FR-A- 1 148 499**
**FR-A- 1 392 804**
**FR-A- 1 450 498**
**US-A- 3 045 047**

**HELVETICA CHIMICA ACTA, vol. 72, no. 173, 1989, pages 1537-1553; C. FEHR et al.: "New aromatic musk odorants: Design and synthesis"**

(73) Titulaire: **FIRMENICH SA**
**1, route des Jeunes**
**CH-1211 Genève 8 (CH)**

(72) Inventeur: **Fehr, Charles**
**6, chemin Ravoux**
**CH-1290 Versoix (CH)**
Inventeur: **Galindo, José**
**7, chemin Croix-du-Levant**
**CH-1220 Les Avanchets (CH)**

(74) Mandataire: **Salvadori, Giuseppe, Dr.**
**c/o Firmenich S.A.**
**Case Postale 239**
**CH-1211 Genève 8 (CH)**

**EP 0 405 427 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

Rank Xerox (UK) Business Services
(3.10/3.09/3.3.3)

## Description

La présente invention a trait au domaine de la parfumerie et, plus particulièrement, à celui des composés qui possèdent des notes odorantes du type musqué.

La recherche de nouveaux corps chimiques à odeur musquée n'a cessé d'augmenter ces dernières années, en raison de la position privilégiée qu'occupe ce type de corps odorants dans la parfumerie moderne. Plusieurs centaines de ces composés sont actuellement connus, constituant une richesse d'information structurale qui a mené certains auteurs à établir des règles qualitatives visant à prévoir le type de structures chimiques qui ont une plus grande probabilité de constituer des composés musqués de bonne qualité, de par l'intensité, la substantivité ou l'élégance et l'individualité de leur note odorante. Si ces règles peuvent permettre de trouver quelques composés plus ou moins intéressants, il n'en est pas moins vrai qu'elles ne remplacent pas l'esprit inventif du chercheur, qui se révèle encore plus pertinent lorsqu'il suit, par intuition, des voies qui seraient en contradiction avec ces principes établis sur la base de l'état de la technique connu. La présente invention en est un exemple.

L'invention concerne des composés nouveaux de formule

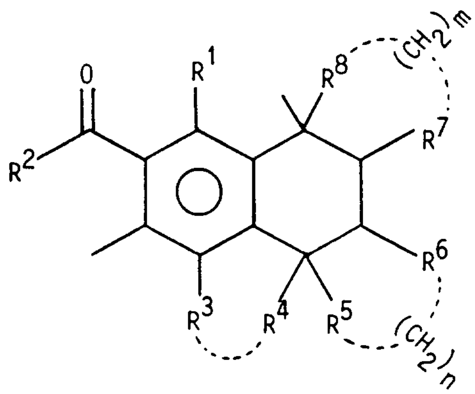

(I)

dans laquelle

a) les indices m et n sont identiques et définissent chacun un nombre entier de valeur 0, les symboles $R^1$ et $R^2$ sont identiques et représentent chacun un atome d'hydrogène, ou sont différents et représentent chacun un atome d'hydrogène ou un radical méthyle, les symboles $R^5$ et $R^8$ représentent chacun un radical méthyle, les symboles $R^6$ et $R^7$, identiques ou différents, représentent chacun un atome d'hydrogène ou un radical méthyle, et soit le symbole $R^4$ représente un radical méthyle et le symbole $R^3$ un atome d'hydrogène ou un radical méthyle, soit les symboles $R^3$ et $R^4$ représentent chacun un radical méthylène faisant partie d'un cycle tel qu'indiqué par la ligne pointillée, les combinaisons suivantes étant cependant exclues :

1. $R^1 = R^2 = R^3 = R^6 = R^7 = H$, ou
2. $R^1 = R^2 = R^3 = H$ et $R^6$ ou $R^7 = CH_3$, ou
3. $R^2 = CH_3$ et $R^3 = R^6 = R^7 = H$, ou
4. $R^2 = CH_3$, $R_1 = R^3 = H$ et $R^6$ ou $R^7 = CH_3$, ou
5. $R^1 = R^3 = CH_3$, ou
6. $R^3 = R^4 = CH_2$ et $R^6$ ou $R^7 = CH_3$;

ou dans laquelle

b) les indices m et n sont différents et définissent chacun un nombre entier de valeur 0 ou 1, le symbole $R^2$ représente un atome d'hydrogène ou un radical méthyle, les symboles $R^1$ et $R^3$ représentent chacun un atome d'hydrogène, le symbole $R^4$ représente un radical méthyle, et soit les symboles $R^5$ et $R^6$ sont identiques (n = 1) et représentent chacun un radical méthylène faisant partie d'un cycle tel qu'indiqué par la ligne pointillée, le symbole $R^7$ représentant un atome d'hydrogène et le symbole $R^8$ un radical méthyle, soit le symbole $R^5$ représente un radical méthyle et le symbole $R^6$ un atome d'hydrogène, les symboles $R^7$ et $R^8$ étant alors identiques (m = 1) et représentant chacun un radical méthylène faisant partie d'un cycle tel qu'indiqué par la ligne pointillée ;

ou tout mélange de deux ou plusieurs isomères structurels de formule (I).

Nous avons découvert que ces composés possèdent des propriétés odorantes fort intéressantes et qu'ils peuvent, de ce fait, être employés, aussi bien en parfumerie fine qu'en parfumerie fonctionnelle, pour la préparation de compositions parfumantes et de produits parfumés. De par la qualité et richesse de leur

note musquée, ils trouvent un emploi de choix dans des bases et concentrés parfumants destinés, en particulier, à la préparation de parfums et eaux de toilette de type masculin, ainsi que de lotions après-rasage. Par ailleurs, ils sont également très appréciés pour le parfumage de savons, gels de douche, shampoings, produits cosmétiques et désodorisants corporels. Ils peuvent encore être employés avantageusement pour le parfumage de détergents et revitalisants textiles, où l'excellente substantivité de leur note musquée garantit un parfumage efficace et durable des tissus traités avec ces produits de lessive.

Les propriétés odorantes des composés selon l'invention apparaissent comme tout à fait surprenantes en vue de l'état de la technique connu, et même contraires aux connaissances acquises dans l'art.

En effet, il est généralement admis que, dans les composés aromatiques musqués dont le cycle benzénique possède un groupe substituant acyle, l'encombrement stérique vis-à-vis de ce groupe fonctionnel conduit à la perte des propriétés odorantes [voir par exemple, M.J. Beets, Structure-Activity Relationships in Human Chemoreception, 207, ASP Ldt., London (1978)]. Cette observation aurait ainsi l'effet de décourager d'emblée toute tentative de substitution supplémentaire du noyau benzénique, dans le squelette de base des composés aromatiques présenté ci-après :

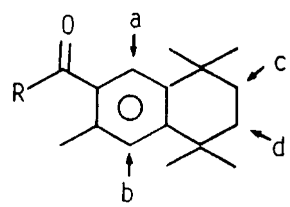

R = H ou $CH_3$

Plusieurs composés musqués obéissant à cette structure de base sont aujourd'hui connus, et son représentant le plus connu est sans doute la Tonalid (marque enregistrée, origine : Polak's Frutal Works Inc.), dont la structure est la suivante

TONALID®

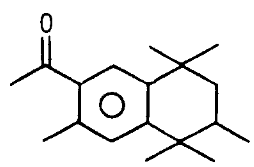

et qui est fort apprécié en parfumerie. Or, nous avons maintenant découvert que des groupes méthyle ou méthylène supplémentaires peuvent être incorporés en position a ou b (voir ci-dessus) du cycle benzénique sans que la perturbation de l'environnement du groupe fonctionnel qui en résulte entraîne une dégradation des propriétés odorantes de ces composés. C'est ainsi que nous avons trouvé plusieurs corps musqués d'excellente qualité, obéissant à la formule (I) définie précédemment.

Par ailleurs, nous avons également trouvé de façon surprenante, que l'incorporation de groupes méthyle ou méthylène en position c et/ou d du même squelette structurel, qui peut s'effectuer simultanément avec la substitution en position a ou b, pouvait conduire à des composés aromatiques musqués nouveaux obéissant à la formule (I) citée et possédant des propriétés odorantes remarquables.

Nous avons constaté que l'incorporation de groupes méthyle ou méthylène supplémentaires dans le squelette de base de ces corps odorants, et dans les positions indiquées, ne semble provoquer que de très légères modifications dans la forme globale de la molécule, contrairement à ce que l'on pourrait prévoir, et donne lieu à des structures plus ou moins sphériques, éminemment compactes et d'une lipophilicité accrue, qui se révèlent être des composés musqués d'une rare puissance, supérieure à celle de leurs analogues connus. Cela veut dire que la substitution alkylique ou alkylénique multiple du squelette de base, notamment dans la partie lipophile de la molécule (positions c et d), peut provoquer des changements marqués et très avantageux dans les propriétés organoleptiques de ces corps odorants, un fait qui n'avait pas été totalement reconnu jusqu'ici.

Un exemple particulièrement intéressant est constitué par les composés selon l'invention de formule

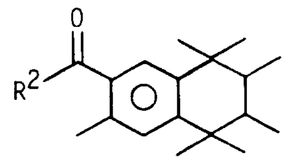

(II)

dans laquelle $R^2$ représente un atome d'hydrogène ou un groupe méthyle. En effet, les deux corps chimiques obéissant à cette formule possèdent des notes odorantes distinctes l'une de l'autre et plus puissantes que celle de la Tonalid (marque enregistrée).

C'est ainsi que le corps aldéhydé se caractérise par une note musquée-ambrée-animale qui évoque le musc naturel. Il possède en plus une note terreuse ressemblant à celle du Cashmeran (6,7-dihydro-1,1,2,3,3-pentaméthyl-4(5H)-indanone; origine : International Flavors & Fragrances Inc.) tout en étant beaucoup plus puissante que cette dernière. Par ailleurs, on y trouve aussi un aspect caractéristique des composés musqués nitro-aromatiques, ce qui rend ce corps d'autant plus intéressant, vu la disparition progressive de ce type de composés de la palette du partumeur. Sa note odorante a non seulement une bonne puissance, bien supérieure à celle des composés musqués existant actuellement sur le marché, mais aussi une remarquable substantivité, comme il ressort des tests qui sont décrits plus loin.

Pour sa part, l'analogue cétonique développe une note odorante très différente, pratiquement libérée du caractère terreux-racineux de celle de l'aldéhyde précité, ce caractère ayant laissé la place à une note musquée plus fine, plus classique, légèrement animale, mais non plus ambrée. Bien que cette note soit moins puissante que celle de l'homologue aldéhyde, elle reste au moins comparable en puissance à celle des meilleurs composés musqués que l'on peut trouver sur le marché, tout en ayant un caractère différent de ceux-ci.

Ces deux corps chimiques de formule (II), désignés 5,6,7,8-tétrahydro-3,5,5,6,7,8,8-heptaméthyl-2-naphtalènecarbaldéhyde et (5,6,7,8-tétrahydro-3,5,5,6,7,8,8-heptaméthyl-2-naphtyl)-1-éthanone, peuvent se présenter sous deux formes isomériques, à savoir, cis et trans. Dans le cas de l'aldéhyde, ces deux formes ont pu être séparées par chromatographie en phase gazeuse et évaluées séparément. Pour le composé cétonique, seul l'isomère trans a pu être séparé du mélange contenant les deux isomères. Ces mélanges des deux isomères, qui peuvent être obtenues lors de la préparation de ces composés, se sont révélées aussi excellentes pour l'utilisation dans la préparation des compositions parfumantes et produits parfumés selon l'invention.

En tant que composés de formule (I) selon l'invention, on citera encore à titre préférentiel, les composés suivants

a) 5,6,7,8-tétrahydro-1,3,5,5,8,8-hexamethyl-2-naphtalènecarbaldéhyde
b) 5,6,7,8-tétrahydro-3,4,5,5,8,8-hexaméthyl-2-naphtalènecarbaldéhyde
c) 5,6,7,8-tétrahydro-1,3,5,5,6,8,8-heptaméthyl-2-naphtalènecarbaldéhyde
d) 5,6,7,8-tétrahydro-3,4,5,5,7,8,8-heptaméthyl-2-naphtalènecarbaldéhyde
e) 5,6,7,8-tétrahydro-1,3,5,5,7,8,8-heptaméthyl-2-naphtalènecarbaldéhyde
f) 5,6,7,8-tétrahydro-3,4,5,5,6,8,8-heptaméthyl-2-naphtalènecarbaldéhyde
g) 1,2,6,7,8,8a-hexahydro-3,6,6,8a-tétraméthyl-4-acénaphtylènecarbaldéhyde
h)(1,2,6,7,8,8a-hexahydro-3,6,6,8a-tétraméthyl-4-acénaphtylényl)-1-éthanone

Du point de vue odorant, tous ces corps chimiques se caractérisent par des notes musquées de puissance et substantivité variées, avec des différences plus ou moins marquées de caractère. Leurs propriétés odorantes seront décrites en détail dans le contexte des exemples de préparation respectifs présentés plus loin.

Par ailleurs lors de la préparation de ces composés on peut également obtenir des mélanges des corps chimiques a) et b), ou c) et d), ou e) et f). Ces mélanges se révèlent aussi parfaitement adéquats pour les applications en parfumerie selon la présente invention.

Finalement, on citera aussi, à titre de composés selon l'invention, les mélanges des composés i) et j) ou k) et l) suivants :

i) 2,3,3a,4,5,9b-hexahydro-5,5,8,9b-tétraméthyl-1H-benz[e]indène-7-carbaldéhyde
j) 2,3,3a,4,5,9b-hexahydro-5,5,7,9b-tétraméthyl-1H-benz[e]indène-8-carbaldéhyde
k) 1-(2,3,3a,4,5,9b-hexahydro-5,5,8,9b-tétraméthyl-1H-benz[e]indén-7-yl)-1-éthanone
l) 1-(2,3,3a,4,5,9b-hexahydro-5,5,7,9b-tétraméthyl-1H-benz[e]indén-8-yl)-1-éthanone.

dont les propriétés organoleptiques seront également décrites en détail dans le contexte des exemples de

préparation respectifs présentés plus loin.

Les proportions dans lesquelles les composés de formule (I) peuvent être utilisés pour obtenir les effets parfumants désirés peuvent varier dans une gamme de valeurs très étendue. L'homme du métier sait par expérience que de telles valeurs dépendent de l'effet particulier recherché, ainsi que de la nature des produits que l'on désire parfumer. On sait également que ces valeurs sont fonction de la nature des autres constituants dans une composition donnée, lorsque l'un des composés selon l'invention est utilisé en tant qu'ingrédient dans une base parfumante ou dans un concentré, en mélange avec d'autres co-ingrédients, des solvants ou des adjuvants usuels.

A titre d'exemple on peut citer des proportions de l'ordre de 5, 10, voire 20% en poids par rapport au poids de la composition, lorsqu'il s'agit de la préparation de bases et concentrés parfumants. Ces valeurs peuvent être bien inférieures lors du parfumage d'articles tels les savons et cosmétiques ou les détergents.

Selon l'invention, on utilisera l'un des composés de formule (I) soit par adjonction directe à l'article que l'on désire parfumer, soit plus généralement en mélange avec d'autres ingrédients parfumants dont l'utilisation est courante en parfumerie. La mention spécifique de tels co-ingrédients est ici superflue. L'état de la technique en présente bien des exemples et l'homme du métier est à même de choisir ceux qui conviendront le mieux à l'effet odorant recherché. On citera comme exemple de référence l'ouvrage de S. Arctander, Perfume and Flavor Chemicals, Montclair, N.J., USA (1969).

Par ailleurs, et tel que précité, on peut également employer les mélanges de composés susmentionnés, ou encore d'autres mélanges de deux ou plusieurs des composés faisant l'objet de l'invention.

A titre d'articles parfumés selon l'invention, on peut citer les parfums, les eaux de toilette et de cologne, notamment de type masculin, ainsi que les lotions après-rasage, les savons et gels de bain et douche, les shampoings, les préparations cosmétiques, les désodorisants et les détergents et revitalisants textiles.

La présente invention concerne également un procédé de préparation des composés de formule (I). En dépit des connaissances acquises dans l'art relatives à la préparation des composés aromatiques musqués, la synthèse des composés selon la présente invention présentait des difficultés particulières dans la mesure où l'on cherchait à synthétiser des molécules stériquement encombrées.

Selon l'invention, le procédé de préparation des composés de formule (I) est caractérisé en ce qu'on

A.

a) fait réagir un composé de formule

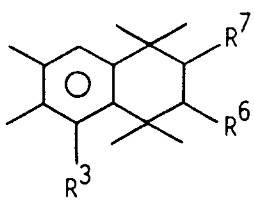

(IIIa)

dans laquelle les symboles $R^3$, $R^6$ et $R^7$, identiques ou différents, représentent chacun un atome d'hydrogène ou un radical méthyle,

avec un agent d'halogénation, sous l'action de la lumière, pour obtenir un mélange d'halogénures de formule

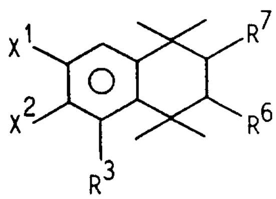

(IV)

dans laquelle les symboles $R^6$ et $R^7$ sont définis comme ci-dessus, le symbole $R^3$ représente un atome d'hydrogène, un atome d'halogène ($X^1 = X^2 = CH_3$) ou un radical méthyle et les symboles $X^1$ et $X^2$ sont identiques et représentent chacun un radical méthyle ($R^3$ = halogène) ou sont différents et représentent chacun un atome d'halogène ou un radical méthyle ($R^3 = H, CH_3$) ;

b) on hydrolyse ensuite ledit mélange d'halogénures pour obtenir un mélange d'alcools correspondants que l'on oxyde par la suite pour obtenir un mélange d'aldéhydes de formule

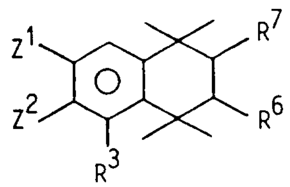

(Ia)

dans laquelle les symboles $R^6$ et $R^7$ sont définis comme ci-dessus, le symbole $R^3$ représente un atome d'hydrogène ou un radical méthyle, ou un groupe CHO lorsque $Z^1 = Z^2 = CH_3$, et les symboles $Z^1$ et $Z^2$ sont identiques et représentent chacun un radical méthyle lorsque $R^3$ = CHO ou sont différents et représentent chacun un groupe CHO ou un radical méthyle lorsque $R^3$ = H, $CH_3$ ;

c) on sépare lesdits aldéhydes et on les traite successivement avec MeLi ou MeMgX (Me = $CH_3$ et X = halogène), $H_2O$ et un agent d'oxydation pour obtenir des cétones de formule

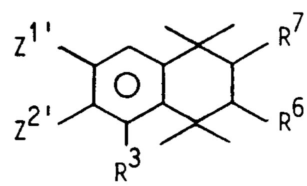

(Ib)

dans laquelle les symboles $R^6$ et $R^7$ ont le sens indiqué ci-dessus, le symbole $R^3$ représente un atome d'hydrogène ou un radical méthyle, ou un groupe $CH_3CO$ lorsque $Z^{1'} = Z^{2'} = CH_3$, et les symboles $Z^{1'}$ et $Z^{2'}$ sont identiques et représentent chacun un radical méthyle lorsque $R^3 = CH_3CO$ ou sont différents et représentent chacun un groupe $CH_3CO$ ou un radical méthyle lorsque $R^3$ = H, $CH_3$ ;

ou

B.

a) fait réagir un composé de formule

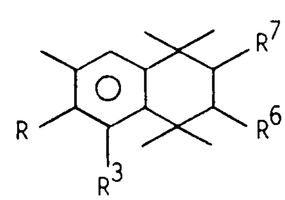

(IIIb)

dans laquelle les symboles R et $R^3$ sont différents et représentent chacun un atome d'hydrogène ou un radical méthyle et les symboles $R^6$ et $R^7$ sont définis ci-dessus,
avec un agent d'oxydation ou de formylation pour obtenir un aldéhyde de formule

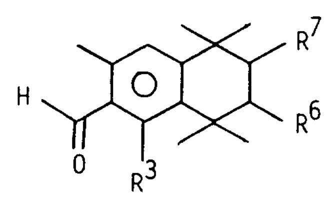

(Ic)

dans laquelle le symbole $R^3$ représente un atome d'hydrogène ou un radical méthyle ;

b) traite ensuite l'aldéhyde de formule (Ic) avec MeLi ou un réactif de Grignard, suivi d'un agent hydrolysant et d'un agent oxydant pour obtenir une cétone de formule

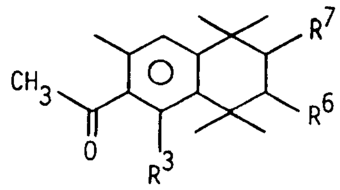

(Id)

dans laquelle les symboles $R^3$, $R^6$ et $R^7$ représentent chacun un atome d'hydrogène ou un radical méthyle;

ou

C. fait réagir un composé de formule

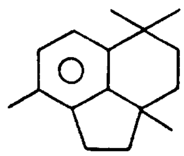

(IIIc)

ou un mélange de composés de formule

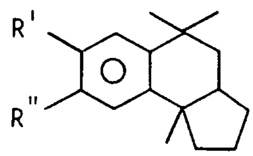

(IIId)

dans laquelle les symboles R' et R'' sont différents et représentent chacun un atome d'hydrogène ou un radical méthyle,

avec $Cl_2CHOCH_3$, dans les conditions de la réaction d'acylation de Friedel-Crafts, pour obtenir respectivement un aldéhyde de formule

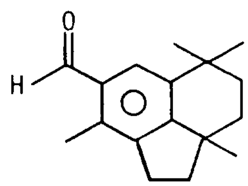

(Ie)

ou un mélange d'aldéhydes de formule

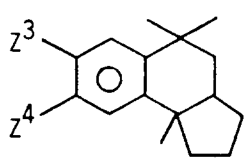

(If)

dans laquelle les symboles $Z^3$ et $Z^4$ sont différents et représentent chacun un groupe CHO ou un radical méthyle ;

ou avec chlorure d'acétyle, en présence d'un acide de Lewis, pour obtenir respectivement une cétone de formule

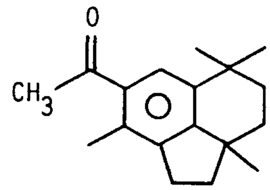

(Ig)

ou un mélange de cétones de formule

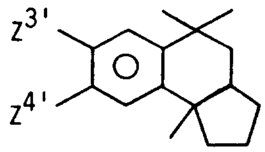

(Ih)

dans laquelle les symboles $Z^{3'}$ et $Z^{4'}$ sont différents et représentent chacun un groupe $CH_3CO$ ou un radical méthyle.

Les produits de départ dans le procédé selon l'invention sont des hydrocarbures de formule

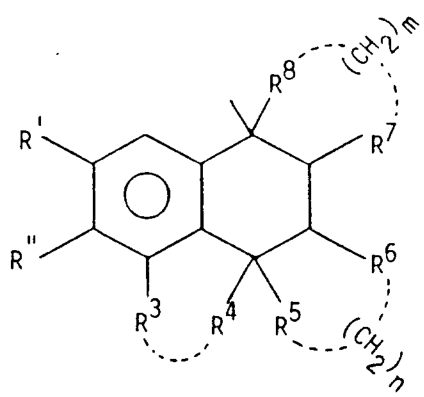

(III)

dans laquelle

a) les indices m et n définissent des nombres entiers identiques de valeur 0, les symboles $R^5$ et $R^8$ représentent chacun un radical méthyle, les symboles $R^6$ et $R^7$, identiques ou différents représentent chacun un atome d'hydrogène ou un radical méthyle, et soit le symbole $R^4$ représente un radical méthyle, les symboles R' et R'' sont identiques et représentent chacun un radical méthyle et le symbole $R^3$ représente un atome d'hydrogène ou un radical méthyle, soit le symbole $R^4$ représente un radical méthyle, le symbole R'' un atome d'hydrogène et les symboles R' et $R^3$ chacun un radical méthyle, soit les symboles $R^3$ et $R^4$ sont identiques et représentent chacun un radical méthylène faisant partie d'un cycle tel qu'indiqué par la ligne pointillée, et les symboles R' et R'' représentent respectivement un atome d'hydrogène et un radical méthyle, la combinaison suivante étant cependant exclue :

1. $R^3 = R^4 = CH_2$ et $R^6$ ou $R^7 = CH_3$ ;

ou dans laquelle

b) les indices m et n sont différents et définissent chacun un nombre entier de valeur 0 ou 1, les symboles R' et R'' représentent respectivement un atome d'hydrogène et un radical méthyle, le symbole $R^3$ représente un atome d'hydrogène, le symbole $R^4$ représente un radical méthyle, et soit les symboles $R^5$ et $R^6$ sont identiques (n = 1) et représentent chacun un groupe méthylène faisant partie d'un cycle tel qu'indiqué par la ligne pointillée, le symbole $R^7$ représentant un atome d'hydrogène et le symbole $R^8$ un radical méthyle, soit le symbole $R^5$ représente un radical méthyle et le symbole $R^6$ un atome d'hydrogène, les symboles $R^7$ et $R^8$ étant alors identiques (m = 1) et représentant chacun un radical méthylène faisant partie d'un cycle tel qu'indiqué par la ligne pointillée,

ou tout mélange de deux ou plusieurs isomères structurels de formule (III).

Ces hydrocarbures peuvent être transformés dans les composés de formule (I) par des méthodes faisant appel à des réactions de type classique, telles que citées précédemment. Plusieurs combinaisons

de ces réactions peuvent être utilisées pour préparer les composés de formule (I), mais on utilisera l'une ou l'autre de ces combinaisons de façon préférentielle, en fonction de la structure du produit que l'on désire obtenir.

C'est ainsi que lorsque l'on veut préparer des composés de structures (Ia) et (Ib), on utilisera comme produit de départ un hydrocarbure de structure (IIIa), que l'on traitera de préférence comme représenté schématiquement ci-après.

SCHEMA I

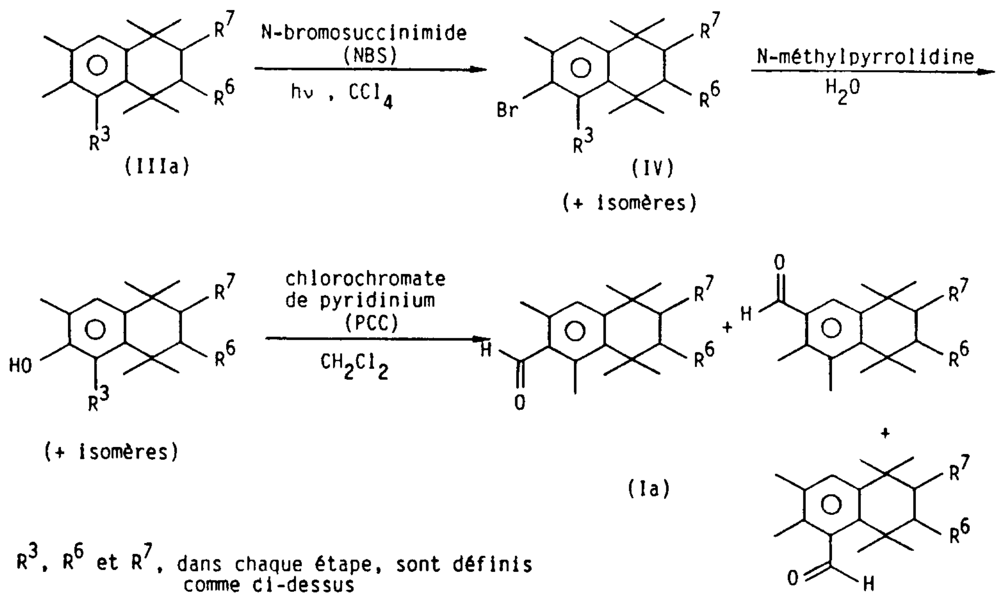


(IIIa) (IV) (+ isomères) (+ isomères) (Ia)

$R^3$, $R^6$ et $R^7$, dans chaque étape, sont définis comme ci-dessus

Les adéhydes de structure (Ia) peuvent être séparés du mélange ainsi obtenu par les méthodes usuelles, par exemple, par chromatographie en phase gazeuse. Ces aldéhydes peuvent également être transformés dans les cétones (Ib) correspondantes par alkylation en présence de MeLi dans l'éther, suivie d'hydrolyse et de l'oxydation de l'alcool ainsi obtenu, par exemple à l'aide de chlorochromate de pyridinium dans le dichlorométhane.

Les produits de départ de formule (IIIa) peuvent être obtenus à partir de dérivés benzéniques, suivant un procédé de synthèse à plusieurs étapes, représenté schématiquement ci-après, et qui fait appel à une combinaison de réactions décrites par T. F. Wood et R. O. Roblin [voir par exemple, T. F. Wood et al, J. Org. Chem. 28, 2248 (1963)] :

SCHEMA II

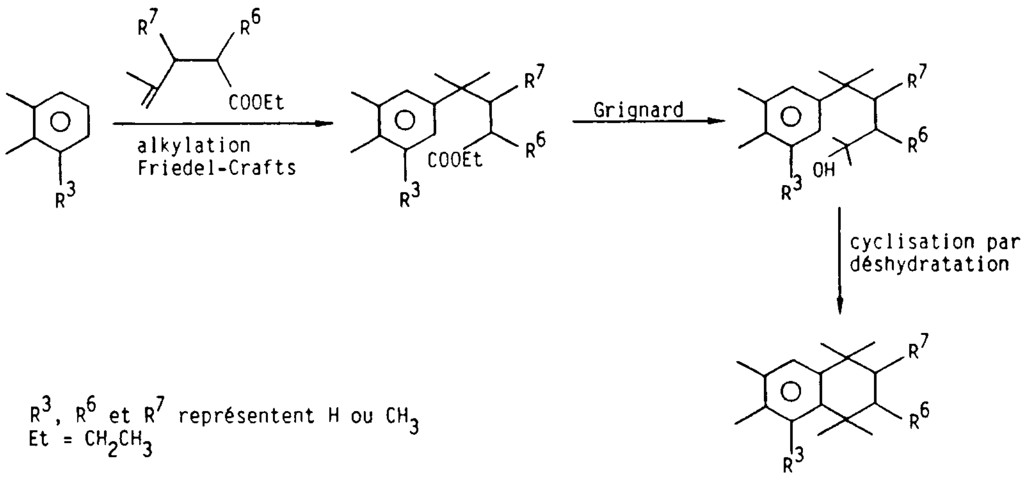


(IIIa)

Typiquement, on utilisera dans la réaction d'alkylation, en tant qu'acide de Lewis, du trichlorure d'aluminium et, en tant que réactif de Grignard dans l'étape suivante, du MeMgI (Me = $CH_3$). Finalement, la réaction de cyclisation est catalysée par un acide, notamment du $H_2SO_4$. Les esters éthyliques utilisés dans la réaction d'alkylation peuvent être obtenus par des procédés classiques, décrits en détail dans le contexte des exemples de préparation présentés plus loin.

Nous avons constaté que lorsque dans la formule (IIIa) $R^6 = R^7 = H$, il était plus avantageux d'utiliser un procédé original, représenté dans le schéma III, pour préparer les précurseurs désirés :

SCHEMA III

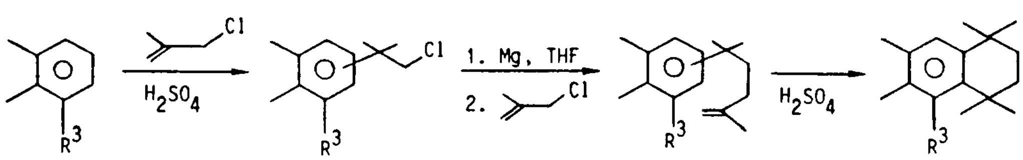


(III a1.)

$R^3$ = H, $CH_3$

Il s'agit d'une séquence, comprenant un couplage entre deux halogénures, suivi d'une alkylation intramoléculaire en milieu acide, qui présente l'avantage d'éviter l'emploi d'un acide de Lewis. Par ailleurs, elle est plus économique et conduit à des résidus plus faibles.

Dans le cas de la préparation des composés de formule (II) selon l'invention et plus particulièrement, de mélanges contenant les deux formes isomériques déjà citées de ces composés, nous avons découvert qu'il était plus avantageux d'utiliser le procédé représenté dans le schéma IV pour obtenir directement le mélange de précurseurs appropries, de structures (IIIb1.) et (IIIb2.)

SCHEMA IV

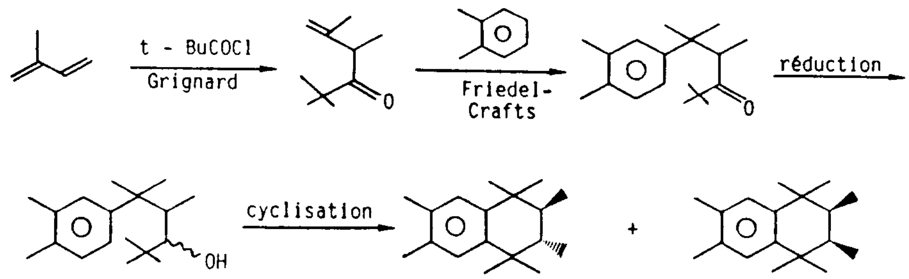


(III b1.) (III b2.)

La première étape de ce procédé consiste en une réaction de Grignard (hydrométallation) à partir de l'isoprène et du chlorure de pivaloyle (t-Bu COCl, Bu = butyle). Il s'agit d'une approche originale qui remplace avantageusement l'acylation de Friedel-Crafts généralement utilisée pour obtenir la cétone illustrée. Il s'ensuit une alkylation Friedel-Crafts classique et une réduction à l'aide de Li $AlH_4$. Alternativement, on pourrait remplacer cette dernière étape par une hydrogénation. L'étape finale de ce procédé consiste en une cyclisation qui peut être réalisée dans des conditions variées. Cependant, la proportion relative des diastéreomères (IIIb1.) et (IIIb2.) dans le mélange obtenu par cette cyclisation dépend fortement des conditions de cette dernière. Par exemple, nous avons constaté qu'il était avantageux d'utiliser l'acide polyphosphorique (PPA) ou du $P_2O_5$ (voir exemple 4) lorsque l'on voulait favoriser la formation du produit (IIIb1.).

La synthèse représentée dans le schéma IV permet ainsi d'accéder à des hydrocarbures symétriques, où les deux groupes méthyle substituants du cycle benzénique sont équivalents, qui peuvent être facilement oxydés au Ce(IV) pour fournir un mélange d'aldéhydes de formule (II), que l'on peut séparer par chromatographie en phase gazeuse. Le mélange de cétones correspondantes peut ensuite être obtenu par des procédés classiques, comme décrit dans l'exemple 6 présenté plus loin.

Cette réaction d'oxydation au Ce(IV) peut en effet être appliquée de façon générale à d'autres hydrocarbures de formule (III).

La synthèse des composés tricycliques de formules (Ie) à (Ih) a été réalisée à partir des hydrocarbures tricycliques (IIIc) et (IIId). Ces derniers peuvent être préparés par des procédés représentés schématiquement ci-dessous et qui font appel à des réactions conventionnelles, dont les conditions sont décrites en détail dans les exemples de préparation respectifs présentés plus loin :

## SCHEMA V

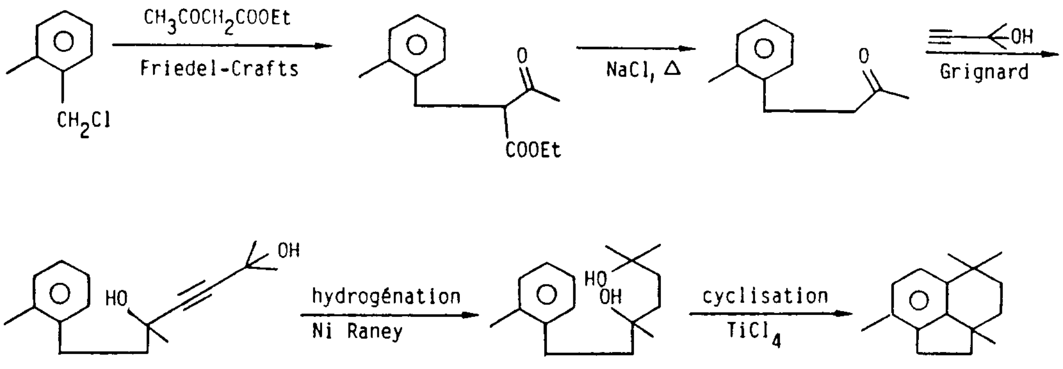


(IIIc)

## SCHEMA VI

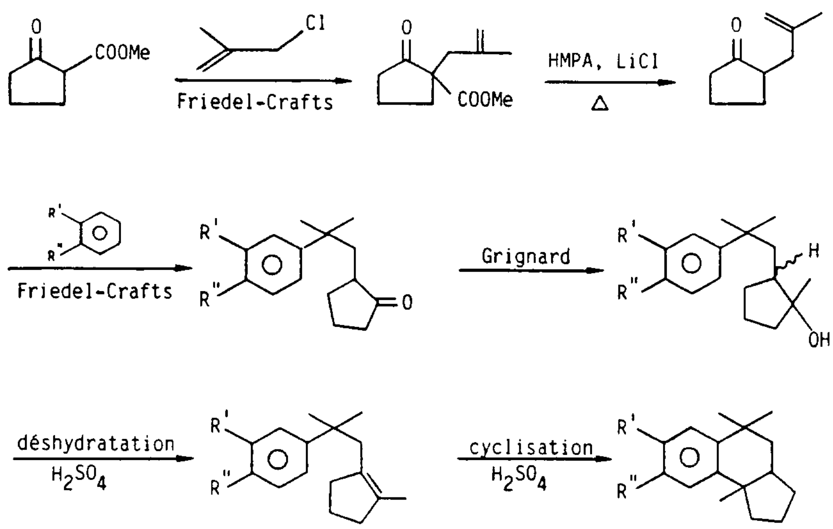


(IIId)
(mélange)

R' = H et R" = $CH_3$, ou vice-versa

L'hydrocarbure (IIIc) peut être transformé en aldéhyde (Ie) et en cétone (Ig) a l'aide de réactions d'acylation du type Friedel-Crafts. De la même façon, on peut obtenir les mélanges de composés de formules (If) ou (Ih) à partir du mélange d'hydrocarbures (IIId). Les conditions spécifiques de ces transformations sont décrites en détail dans les exemples 7 à 10 présentés plus loin.

L'invention sera maintenant décrite plus en détail dans les exemples de préparation suivants, dans lesquels les températures sont présentées en degrés centigrades et les abréviations ont le sens usuel dans l'art.

Par ailleurs, des exemples d'application des composés selon l'invention en parfumerie sont également décrits en détail. Ces exemples représentent des modes d'exécution préférentiels de l'invention, mais ne doivent pas être perçus comme étant limitatifs de cette dernière. En particulier et suivant les effets parfumants désirés, des composes de formule (I) autres que ceux cités dans les exemples décrits, pourront être utilisés à titre d'ingrédients parfumants dans les compositions parfumantes et articles parfumés selon l'invention.

Exemple 1

Préparation de 5,6,7,8-tétrahydro-1,3,5,5,8,8-hexaméthyl-2-naphtalènecarbaldéhyde et de 5,6,7,8-tétrahydro-3,4,5,5,8,8-hexaméthyl-2-naphtalènecarbaldéhyde

a) Préparation de 1,2,3,4-tétrahydro-1,1,4,4,5,6,7-heptaméthylnaphtalène

Dans un ballon à 3 cols de 3 l équipé d'une agitation mécanique et maintenu sous azote, on a additionné lentement (2 h) du chlorure de méthallyle (426,0 g) à un mélange de 1,2,3-triméthylbenzène (1582,0 g) et $H_2SO_4$ (84,0 g), en maintenant la température à 20°. Après 3 h, on a décanté le $H_2SO_4$ et lavé la phase organique avec, successivement, de l'eau, une solution aqueuse saturée de $NaHCO_3$ et une solution aqueuse saturée de NaCl. On a récupéré l'excès de 1,2,3-triméthylbenzène (1100,0 g) par distillation (70°/9,31x$10^3$ à 2,66x$10^3$ Pa) et distillé le résidu (97-100°/2,66 Pa) pour obtenir 726,0 g d'un mélange de deux isomères (A/B : env. 40/60) dont les données analytiques sont présentées ci-après. Ce mélange a été utilisé tel quel dans la réaction suivante :

A. 1-(2-chloro-1,1-diméthyléthyl)-3,4,5-triméthylbenzène

IR ($CDCl_3$) : 2950, 1485, 1390 $cm^{-1}$

RMN ($^1$H, 60MHz) : 1,38(s, 6H); 2,13(s, 3H); 2,27(s, 6H); 3,59(s,2H); 7,01(s,2H) delta ppm

SM :210($M^+$, 8), 174(7), 161(100), 133(28), 121(34), 115(14), 105(16), 91(19),77(14).

B. 1-(2-chloro-1,1-diméthyléthyl)-2,3,4-triméthylbenzène

IR ($CDCl_3$) : 2950, 1485, 1390 $cm^{-1}$

RMN ($^1$H, 60MHz) : 1,50(s, 6H); 2,17(s, 3H); 2,27(s, 3H); 2,38(s, 3H); 3,82(s,2H); 6,96(d, J=8Hz, 1H); 7,13(d, J=8Hz, 1H) delta ppm

SM :210($M^+$, 10), 174(12), 161(100), 133(56), 121(34), 115(19), 105(22), 91(27),77(19).

Dans un ballon à sulfuration de 1,5 l, équipé d'une agitation mécanique et maintenu sous azote, on a chauffé à reflux une suspension de Mg (41,0 g) dans du THF (tétrahydrofuranne, 100 ml). On a ajouté ensuite 10 ml d'une solution du mélange (A+B) préparé ci-dessus (300,0 g) dans du THF (100 ml). Dès que la réaction a démarré, on a ajouté du THF (300 ml) et, ensuite, le restant du mélange (A+B) dans du THF (75 min). Le mélange réactionnel a été agité pendant 30 min en maintenant la température à 75°. Par la suite, on a additionné du chlorure de méthallyle (193,0 g) pendant 20 min, en maintenant le mélange de la réaction à reflux. Pendant l'introduction, on a observé la précipitation de $MgCl_2$ qui a alourdi le mélange mais sans gêner l'agitation. Après 30 min, on a refroidi le mélange réactionnel à 10° et hydrolysé en ajoutant de l'eau (400 ml). On a séparé les phases et extrait la phase aqueuse à l'éther. Les phases organiques combinées ont été lavées avec une solution aqueuse saturée de NaCl et les solvants évaporés. Par distillation (130-135°/2,66x$10^2$ Pa) on a obtenu 294,0 g (rend. 90%) d'un mélange huileux incolore dont le spectre RMN ($^1$H, 60MHz) montrait un singulet élargi, à 4,65 delta ppm. Ce mélange a été employé directement dans la réaction de cyclisation suivante.

Dans un ballon à 3 cols de 500 ml, équipé d'une agitation mécanique et maintenu sous azote, on a ajouté 288,0 g du mélange obtenu en dernier lieu à un mélange d'éther de pétrole 30-50° (100 ml) et $H_2SO_4$ (7,0 g), pendant 1 h, en maintenant la température à 5°-10°. Après 30 min à 10°, on a séparé le $H_2SO_4$ de la phase organique et lavé cette dernière avec successivement $H_2O$, une solution saturée de $NaHCO_3$ et une solution saturée de NaCl. Par recristallisation du produit brut dans l'éthanol (1,1 l), on a obtenu 240,0 g (rend. 83%) de 1,2,3,4-tétrahydro-1,1,4,4,5,6,7-heptaméthylnaphtalène.

P. fusion 79-82°

IR ($CDCl_3$) : 2920, 1455, 1395, 1380 $cm^{-1}$

RMN ($^1$H, 60MHz) : 1,27(s, 6H); 1,41(s, 6H); 1,65(s, 4H); 2,12(s, 3H); 2,27(s,3H) ; 2,38(s, 3H) ; 7,01(s, 1H) delta ppm.

SM :230($M^+$, 22), 216(15), 215(100), 174(11), 173(73), 171(14), 159(41), 141(10),128(10), 57(16).

b) Dans un ballon à sulfuration, équipé d'une agitation mécanique, d'un réfrigérant et d'une entrée d'azote, on a dissous 6,25 g du 1,2,3,4-tétrahydro-1,1,4,4,5,6,7-heptaméthylnaphtalène préparé en a) dans du $CCl_4$ (70 ml) et on a ajouté du NBS (N-bromosuccinimide, 5,56 g). On a irradié la suspension avec une lampe de 100 W, ce qui a porté le mélange réactionnel à reflux. Après 45 min, on a laissé revenir à la température ambiante, versé sur $H_2O$, et extrait trois fois à l'éther. Les phases organiques réunies ont été lavées avec une solution aqueuse saturée de NaCl, séchées sur $Na_2SO_4$, filtrées et les solvants évaporés.

Le mélange brut ainsi obtenu (10,5 g) était constitué par les trois bromures dérivés de la substitution du cycle benzénique (voir schéma I) et contenait environ 15% en poids non réagis du composé naphtalénique de départ, car la réaction n'a pas été complétée afin d'empêcher la formation de dibromures. Ce mélange brut a ensuite été dissous dans N-méthyl-pyrrolidone (70 ml) et $H_2O$ (10 ml) et chauffé à reflux

pendant 1 h. On a laissé refroidir le mélange réactionnel à 20° et extrait à l'éther. La phase organique a été lavée à l'eau trois fois, ensuite avec une solution aqueuse saturée de NaCl, séchée sur $Na_2SO_4$, et les solvants on été évaporés pour fournir 7,65 g de produit brut. La chromatographie sur colonne de $SiO_2$ (200 g), en utilisant comme éluant un mélange cyclohexane/éther 98:2, a permis d'obtenir une fraction non-polaire contenant le 5,6,7,8-tétrahydro-2,3,5,5,8,8-hexaméthyl-1-naphtalèneméthanol (1,3 g) et une fraction polaire contenant les isomères 5,6,7,8-tétrahydro-1,3,5,5,8,8-hexaméthyl-2-naphalèneméthanol et 5,6,7,8-tétrahydro-3,4,5,5,8,8-hexaméthyl-2-naphtalèneméthanol (1,86 g, respectivement 80:20). Le rendement de ces deux fractions combinées était de 3,16 g (47% en alcools).

Dans un ballon à 3 cols de 100 ml équipé d'une agitation magnétique, d'un thermomètre et d'une entrée d'azote, on a dissous du PCC (chlorochromate de pyridinium, 2,18 g) dans du chlorure de méthylène (15 ml) et on a ajouté, goutte à goutte, en maintenant la température à 20°, une solution de la fraction polaire sus-mentionnée (1,55 g, 4:1) dans le chlorure de méthylène (5 ml). Deux heures plus tard on a filtré le mélange de la réaction, qui était devenu brun foncé, sur $SiO_2$ (20 g) avec $CH_2Cl_2$, et évaporé le solvant. Après recristallisation dans du méthanol, on a obtenu 501 mg d'un mélange contenant 5,6,7,8-tétrahydro-1,3,5,5,8,8-hexaméthyl-2-naphtalènecarbal-déhyde et 5,6,7,8-tétrahydro-3,4,5,5,8,8-hexaméthyl-2-naphtalènecarbal-déhyde, dans les proportions respectives de 9:1, et 439,0 mg d'eaux-mères (contenant 85% des deux aldéhydes sus-mentionnés). Le rendement dans ledit mélange contenu dans la fraction de recristallisation et dans les eaux-mères était de 57%. La chromatographie en phase préparative a permis d'obtenir un échantillon de 5,6,7,8-tétrahydro-3,4,5,5,8,8-hexaméthyl-2-naphtalènecarbaldéhyde contenant 10% de 5,6,7,8-tétrahydro-1,3,5,5,8,8-hexaméthyl-2-naphtalènecarbal-déhyde. Les données analytiques de ces composés sont présentées ci-après.

Un traitement du 5,6,7,8-tetrahydro-2,3,5,5,8,8-hexaméthyl-1-naphtalèneméthanol (1,30 g) avec PCC, de façon analogue à celle décrite ci-dessus, à permis d'obtenir 456,0 mg de 5,6,7,8-tétrahydro-2,3,5,5,8,8-hexaméthyl-1-naphtalènecarbaldéhyde (P. fusion 74-78°) et des eaux-mères (476,0 g, 80% pur), avec un rendement estimé à 61%. Les données analytiques de ce produit sont également présentées ci-après.

5,6,7,8-tétrahydro-1,3,5,5,8,8-hexaméthyl-2-naphtalènecarbaldéhyde

IR ($CDCl_3$) : 2975, 2940, 2850, 1685, 1600, 1385 $cm^{-1}$

RMN ($^1H$, 360MHz) : 1,30(s, 6H); 1,45(s, 6H); 1,68(large s, 4H); 2,47(s, 3H) ;2,70(s, 3H); 7,07(s, 1H); 10,58(s, 1H) delta ppm.

SM : 244($M^+$, 50), 229(100), 187(19), 173(22), 159(56), 145(13), 128(10).

Ce composé possédait une jolie note musquée, nettement ambrette (graines).

5,6,7,8-tétrahydro-3,4,5,5,8,8-hexaméthyl-2-naphtalènecarbaldéhyde

IR ($CDCl_3$) : 2975, 2940, 2850, 1685, 1600, 1385 $cm^{-1}$

RMN ($^1H$, 360MHz) : 1,33(s, 6H); 1,47(s, 6H); 1,68(s, 4H); 2,42(s, 3H); 2,53(s, 3H); 7,67(s, 1H); 10,26-(s, 1H) delta ppm.

SM : 244($M^+$, 50), 229(100), 187(19), 173(22), 159(56), 145(13), 128(10).

Ce composé possédait une note musquée.

5,6,7,8-tétrahydro-2,3,5,5,8,8-hexaméthyl-1-naphtalènecarbaldéhyde

IR ($CDCl_3$) : 2990, 2955, 2890, 1705, 1470, 1380 $cm^{-1}$

RMN ($^1H$, 360MHz) : 1,28(s, 6H); 1,36(s, 6H); 1,61-1,72(m, 4H); 2,16(s, 3H); 2,26(s, 3H); 7,20(s, 1H); 10,83(s, 1H) delta ppm.

SM : 244($M^+$, 23), 229(100), 211(40), 196(18), 185(15), 169(17), 159(29), 141(17), 128(15), 115(15).

c) Le 5,6,7,8-tétrahydro-1,3,5,5,8,8-hexaméthyl-2-naphtalènecarbaldéhyde a également été préparé de façon sélective, à partir de 1,2,3,4-tétrahydro-1,1,4,4,5,7-hexaméthylnaphtalène.

Ce dernier a été obtenu suivant un procédé de préparation analogue à celui décrit en a), en utilisant comme produits de départ du m-xylène (490,0 g), du chlorure de méthallyle (150,0 g) et $H_2SO_4$ (30,0 g), dont la réaction a permis d'obtenir 182,8 g (rend. 56%) de 1-(2-chloro-1,1-diméthyl)-2,4-diméthylbenzène (contenant 10% de régioisomère). 100,0 g de ce dernier composé ont ensuite été traités dans les conditions décrites en a), pour fournir 97,0 g (rend. 88%) de 1-(1,1,4-triméthyl-4-pentényl)-2,4-diméthylbenzène. La réaction de cyclisation de ce dernier produit (86,9 g) a fourni 80,6 g (rend. 93%) de 1,2,3,4-tétrahydro-1,1,4,4,5,7-hexaméthyl-naphtalène.

P. Eb. 120°/2,66x$10^3$ Pa.

IR : 2910, 1600, 1455, 1390, 1375 $cm^{-1}$

RMN ($^1H$, 60MHz) : 1,24(s, 6H); 1,35(s, 6H); 1,66(s, 4H); 2,23(s, 3H); 6,75(large s, 1H); 7,00(large s, 1H) delta ppm.

SM : 216($M^+$, 33), 201(100), 159(67), 145(29), 141(13), 128(11), 115(10).

Un mélange de 1,2,3,4-tétrahydro-1,1,4,4,5,7-hexaméthylnaphtalène (5,0 g) et $TiCl_4$ (7,32 g) dans le chlorure de méthylène (40 ml) a été traité avec $Cl_2CHOCH_3$ (2,66 g) dans le chlorure de méthylène (5 ml), à 0° pendant 20 min. On a laissé la température du mélange de la réaction atteindre 20° (20 min), versé sur de l'eau glacée et extrait à l'éther. La phase organique a été lavée successivement avec une solution aqueuse à 10% de NaOH, de l'eau et une solution aqueuse saturée de NaCl, séchée sur $Na_2SO_4$, évaporée et recristallisée dans le méthanol pour fournir 4,06 g (rend. 72%) de 5,6,7,8-tétrahydro-1,3,5,5,8,8-hexaméthyl-2-naphtalène-carbaldéhyde, dont les données étaient identiques à celles du produit décrit en b).

Exemple 2

Préparation de 5,6,7,8-tétrahydro-1,3,5,5,6,8,8-heptaméthyl-2-naphtalènecarbaldéhyde et de 5,6,7,8-tétrahydro-3,4,5,5,7,8,8-heptaméthyl-2-naphtalène carbaldéhyde

a) Préparation de 3,4-diméthyl-4-penténoate d'éthyle

Dans un ballon de 2 l équipé d'une agitation mécanique et maintenu sous azote, on a ajouté une solution d'acide tiglique (100,0 g) dans l'éther sulfurique (300 ml) a une suspension de $LiAlH_4$ (28,5 g) dans l'éther (300 ml), pendant 3 h, en maintenant la température à 5°. Le mélange de réaction a été porté à reflux pendant 1 h, et ensuite laissé sous agitation, à température ambiante, pendant une nuit. Le mélange a été refroidi dans un bain glacé et on lui a ajouté, goutte à goutte, 100 ml de HCl à 5%, puis 600 ml de HCl à 15%, ainsi que 200 ml d'éther pour éviter que le mélange prenne en masse. Le mélange réactionnel a été extrait à l'éther sulfurique (3x400 ml) et les extraits lavés successivement avec une solution saturée de NaCl (3x50 ml), de $Na_2CO_3$ à 10% (20 ml) et avec de l'eau. Les phases éthérées ont été réunies, séchées sur $Na_2SO_4$ et concentrées par évaporation (40°/9,7x10$^4$ Pa). Le résidu (84,8 g) a été fractionné dans une colonne Vigreux sous vide ordinaire pour fournir 59,3 g (rend. 69%) de 2-méthyl-2-butén-1-ol.

Dans un ballon de 3000 ml équipé d'une agitation mécanique et d'un réfrigérant, et maintenu sous azote, on a chauffé un mélange de triéthylorthoacétate (1117,8 g), acide propionique (2,3 g) et 2-méthyl-2-butén-1-ol (59,3 g) pendant 72 h à 118° pour faire distiller l'éthanol au fur et à mesure qu'il se formait. L'excès de triéthylorthoacétate a été récupéré et la distillation a été terminée sous pression réduite. Le produit brut obtenu (69,0 g) contenait environ 80% du penténoate désiré. La purification sur colonne Fischer a fourni 43,7 g (41%) de 3,4-diméthyl-4-penténoate d'éthyle.

b) Préparation de 3,4-diméthyl-4-(3,4,5-triméthyl-1-phényl)pentanoate d'éthyle

Dans un ballon à sulfuration équipé d'une agitation mécanique, d'un thermomètre, d'une ampoule d'introduction et d'une entrée d'azote, on a ajouté, goutte à goutte, pendant 1 h, 52,0 g du 3,4-diméthyl-4-penténoate d'éthyle préparé selon a) à une suspension de $AlCl_3$ (115,04 g) dans le 1,2,3-triméthylbenzène (359,99 g), en maintenant la température à 0-5°. Dès la fin de l'introduction on a laissé la température remonter à 20° et après 15 min versé sur de l'eau glacée. Le mélange a été extrait à l'éther et lavé avec, successivement, du NaOH à 5%, de l'eau et une solution saturée de NaCl. On a séché sur $Na_2SO_4$, filtré et évaporé les solvants. L'excès de 1,2,3-triméthylbenzène a été distillé à 70°/2,66x10$^3$ Pa. Le résidu a été distillé à 160°/2,66x10$^2$ Pa pour fournir 70,23 g (rend. 76%) de 3,4-diméthyl-4-(3,4,5-triméthyl-1-phényl)-pentanoate d'éthyle.

IR : 2970, 1740, 1455, 1380, 1305, 1190 cm$^{-1}$

RMN ($^1$H, 60MHz) : 0,85(d, J = 7Hz, 3H); 1,23(t, J = 7Hz, 3H); 1,23(s, 6H); env.1,80(m, 1H); env.2,20-(m, 2H); 2,15(s, 3H); 2,26(s, 6H); 4,06(q,J = 7Hz, 2H); 6,93(s, 2H) delta ppm.

SM : 276(M$^+$, 3), 231(3), 161(100), 147(8), 133(14), 121(13), 105(7), 91(6).

c) Préparation de 2,4,5,5-tétraméthyl-5-(3,4,5-triméthyl-1-phényl)-2-pentanol

Dans un ballon à sulfuration de 1,5 l équipé d'un réfrigérant, d'une ampoule d'introduction et d'une entrée d'azote on a introduit 15,12 g de Mg et recouvert celui-ci d'éther anhydre (20 ml). On a amorcé la réaction de Grignard par addition de 5 à 10 ml d'une solution de MeI (96,56 g) dans l'éther (180 ml). Dès que la réaction a démarré (reflux d'éther), on a ajouté au mélange réactionnel une solution du pentanoate préparé en b) (69,0 g) dans l'éther (200 ml). On a poursuivi l'addition de la solution de MeI susmentionnée en contrôlant le reflux d'éther avec un bain d'eau froide (durée de l'addition environ 1 h). On a laissé réagir pendant 1 h en maintenant la température à 20°, puis hydrolysé prudemment avec de l'eau glacée. Le mélange de la réaction a été extrait à l'éther, lavé avec une solution saturée de NaCl, séché sur $Na_2SO_4$, filtré et les solvants évaporés. On a obtenu 65,1 g (rend. env. 100%) de l'alcool désiré, qui a été utilisé tel quel dans la réaction de cyclisation suivante.

d) Préparation de 1,2,3,4-tétrahydro-1,1,2,4,4,5,6,7-octaméthylnaphtalène

Dans un ballon de 250 ml à 3 cols, équipé d'une agitation mécanique, d'un thermomètre, d'une ampoule d'introduction et d'une entrée d'azote, on a introduit 100 g de $H_2SO_4$ à 90% et ajouté goutte à goutte (1 h) une solution de l'alcool brut préparé en c) (65,1 g) dans l'éther de pétrole 80-100° (environ 50 ml), en maintenant la température entre 0 et 10°. Dès la fin de l'introduction, on a laissé la temperature remonter à 20° et, après 30 min, décanté le $H_2SO_4$, puis ajouté de l'eau glacée (environ 300 ml). On a extrait à l'éther, lavé avec NaOH à 10% et NaCl saturé, séché sur $Na_2SO_4$, filtré et évaporé les solvants. La recristallisation du produit brut dans l'éthanol a fourni 37,3 g de 1,2,3,4-tétrahydro-1,1,2,4,4,5,6,7-octaméthylnaphtalène et 18,77 g d'eaux-mères contenant environ 55% de ce même composé (rend. total 79%). Ce composé a été employé comme produit de départ dans la synthèse des aldéhydes désirés.

IR ($CDCl_3$) : 2920, 1455, 1385, 1360 $cm^{-1}$

RMN ($^1H$, 60MHz) :0,94(d, J=7Hz, 3H); 1,10(s, 3H); 1,25(s, 3H); 1,39(s, 3H) ;1,43(s, 3H); 1,60-1,90-(m, 3H); 2,11(s, 3H); 2,23(s, 3H); 2,36(s,3H) ;7,07(s, 1H) delta ppm.

SM : 244($M^+$, 30), 229(100), 187(92), 173(73), 156(16), 141(17), 128(12), 115(11),57(13), 41(14).

e) On a procédé selon l'exemple 1b) (schéma I).

Dans la réaction d'halogénation on a utilisé les quantités et réactifs suivants : NBS(28,82 g), 1,2,3,4-tétrahydro-1,1,2,4,4,5,6,7-octaméthyl-naphtalène (préparé en d), 35 g) et $CCl_4$ (350 ml). On a obtenu après traitement 54,1 g de produit brut, constitué par le mélange de bromures benzyliques et de l'octaméthylnaphtalène de départ non-réagi.

Ce produit brut (54,1 g) a été utilise dans la réaction d'hydrolyse, avec N-méthylpyrrolidone (300 ml) et $H_2O$ (45 ml). Après le traitement on a obtenu 44,0 g de produit brut contenant les 5,6,7,8-tétrahydro-3,4,5,5,7,8,8-heptaméthyl-2-naphtalèneméthanol, 5,6,7,8-tétrahydro-1,3,5,5,6,8,8-heptaméthyl-2-naphtalèneméthanol et 5,6,7,8-tétrahydro-2,3,5,5,6,8,8-heptaméthyl-1-naphtalèneméthanol, ainsi qu'environ 15% de 1,2,3,4-tétrahydro-1,1,2,4,4,5,6,7-octaméthylnaphtalène non réagi.

Ce produit brut (44,0 g, environ 77% en poids d'alcools) a été utilisé dans la réaction d'oxydation, avec PCC (44,9 g) et $CH_2Cl_2$ (300 ml). Après la filtration on a obtenu deux fractions dont une contenait 5,22 g de l'octaméthylnaphtalène susmentionné et la deuxième contenait 11,8 g d'un mélange constitué par 5,6,7,8-tétrahydro-1,3,5,5,6,8,8-heptaméthyl-2-naphta-lènecarbaldéhyde, 5,6,7,8-tétrahydro-3,4,5,5,7,8,8-heptaméthyl-2-naphtalène-carbaldéhyde et 5,6,7,8-tétrahydro-2,3,5,5,6,8,8-heptaméthyl-1-naphtalène-carbaldéhyde dans les proportions respectives de 4:5:1. (rend. sur 3 étapes 27%). Par chromatographie en phase préparative, on a obtenu un mélange 9:1 des deux premiers aldéhydes.

5,6,7,8-tétrahydro-1,3,5,5,6,8,8-heptaméthyl-2-naphtalènecarbaldéhyde

IR ($CDCl_3$) : 2920, 1680, 1595, 1460, 1370 $cm^{-1}$

RMN ($^1H$, 360MHz) : 0,99(d, J=7Hz, 3H); 1,16(s, 3H); 1,28(s, 3H); 1,35(dd,J=14,2Hz, 1H); 1,42(s, 3H); 1,48(s, 3H); 1,67(t, J=14Hz, 1H); 1,86(m,1H); 2,49(s, 3H); 2,72(s, 3H); 7,12(s, 1H); 10,61(s, 1H) delta ppm.

SM : 258($M^+$, 35), 245(95), 201(47), 187(60), 173(100), 159(35), 141(20), 128(17),115(13), 91(13), 57-(18), 41(15).

5,6,7,8-tétrahydro-3,4,5,5,7,8,8-heptaméthyl-2-naphtalènecarbaldéhyde

IR ($CDCl_3$) : 2920, 1680, 1595, 1460, 1370 $cm^{-1}$

RMN ($^1H$, 360MHz) : 2,43(s, 3H); 2,52(s, 3H); 7,73(s, 1H); 10,61(s, 1H) delta ppm.

SM : 258($M^+$, 35), 245(95), 201(47), 187(60), 173(100), 159(35), 141(20), 128(17), 115(13), 91(13), 57-(18), 41(15).

Ce mélange possédait un jolie note musquée, propre mais un peu faible.

## Exemple 3

Préparation de 5,6,7,8-tétrahydro-1,3,5,5,7,8,8-heptaméthyl-2-naphtalènecarbaldéhyde et 5,6,7,8-tétrahydro-3,4,5,5,6,8,8-heptaméthyl-2-naphtalènecarbaldéhyde

a) Préparation de 2,4-diméthyl-4-penténoate d'éthyle

Dans un ballon de 3000 ml, équipé d'un agitateur mécanique, d'un thermomètre, d'un réfrigérant, et maintenu sous azote, on a préparé une solution d'éthylate de sodium (46,0 g Na, 700 ml éthanol absolu) à laquelle on a ajouté 286,0 g d'acétoacétate d'éthyle, pendant 30 min, en maintenant la température à 15°. On a laissé le mélange sous agitation pendant 30 min à température ambiante et ensuite introduit 182,0 g de chlorure de bêta-méthallyle, à température ambiante et en une seule fois. On a laissé agiter pendant environ 50 h et porté à reflux pendant 1 h. Le précipité de chlorure de sodium a été filtré et le

filtrat concentré par évaporation des solvants. Le résidu (402,6 g) a été fractionné sur colonne Vigreux, puis dans une colonne remplie d'hélices de verre surmontée d'une tête de colonne à reflux total. On a obtenu 161,4 g de 2-acétyl-4-méthyl-4-penténoate d'éthyle que l'on a utilisé dans la réaction suivante.

Dans un ballon à trois cols de 2000 ml, équipé d'une agitation mécanique, d'un réfrigérant, et maintenu sous azote, on a introduit 24,3 g de Na et 400 ml d'éthanol pour préparer l'éthylate de sodium. On a refroidi la solution à 15° et ajouté le penténoate préparé précédemment (161,4 g), pendant 30 min et en maintenant la température à 15-20°. On a ensuite ajouté 150,0 g de iodure de méthyle en une seule fois. La réaction exothermique a été contrôlée à l'aide d'un bain glacé de façon à maintenir la température à 30°, ceci pendant environ 90 min. On a laissé sous agitation pendant 2 h 30 min à 20° et ensuite porté à reflux pendant 4 h. Le mélange de la réaction a été laissé en repos pendant 56 h et ensuite on a filtré le précipité de NaI. 700 ml de toluène ont été ajoutés et le mélange a été de nouveau filtré. On a distillé 700 ml d'azéotrope toluène/éthanol (rotavapeur, 74°C/6x$10^4$ Pa) et rajouté 250 ml au résidu. Après refroidissement à 5° on a de nouveau filtré. Le filtrat a été concentré sur rotavapeur (74°/2,7x$10^4$ Pa). On a obtenu 240,3 g de produit brut, qui a été rectifié dans une colonne Vigreux et, ensuite dans une colonne remplie d'hélices de verre surmontée d'une tête de colonne à reflux total. On a obtenu 78,1 g de 2-acétyl-2,4-diméthyl-4-penténoate d'éthyle pur que l'on a utilisé dans la réaction suivante.

b) Préparation de 2,4-diméthyl-(3,4,5-triméthyl-1-phényl)pentanoate d'éthyle

On a procédé comme dans l'exemple 2b) en utilisant les quantités et réactifs suivants : $AlCl_3$ (125,2 g), 1,2,3-triméthylbenzène (390,9 g), 2,4-diméthyl-4-penténoate d'éthyle (préparé selon a), 56,5 g). Après traitement et distillation on a obtenu 81,9 g du produit désiré (P. Eb. 160°/2,7x$10^2$ Pa, rend. 82%).

IR : 2950, 1730, 1450, 1370, 1180, 1150 $cm^{-1}$

RMN ($^1H$, 60MHz) : 1,02(d, J=7Hz, 3H); 1,14(t, J=7,5Hz, 3H); 1,24(s, 6H) ;env.1,70(m, 2H); 2,12(s, 3H); 2,27(s, 6H); env.3,0-3,5(m, 1H); 3,91(q,J=7,5Hz, 2H); 6,93(s, 2H) delta ppm.

SM : 276($M^+$, 6), 231(5), 187(5), 161(100), 133(11), 121(10), 105(5).

c) Préparation de 2,3,5,5-tétraméthyl-5-(3,4,5-triméthyl-1-phényl)-2-pentanol

On a procédé comme dans l'exemple 2c), en utilisant les quantités et réactifs suivants : Mg (17,7 g), MeI (108,9 g), 2,4-diméthyl-(3,4,5-triméthyl-1-phényl)pentanoate d'éthyle (préparé selon b), 81,5 g), éther (650 ml). On a obtenu 82,0 g de produit brut qui a été utilisé tel quel dans la réaction de cyclisation suivante.

d) Préparation de 1,2,3,4-tétrahydro-1,1,2,4,4,6,7,8-octaméthylnaphtalène

On a procédé comme dans l'exemple 2d), en utilisant les quantités et réactifs suivants : alcool brut préparé en c) (82 g), $H_2SO_4$ à 90% (100 g), éther de pétrole (env. 50 ml). Après la cristallisation, on a obtenu 50 g du produit désiré pur et 24,3 g d'eaux-mères contenant environ 60% de ce même produit (rend. global 68%). Ce dernier a été utilisé comme produit de départ dans la synthèse des aldéhydes désirés.

IR ($CDCl_3$) : 2950, 1450, 1380, 1360 $cm^{-1}$

RMN ($^1H$, 60MHz) : 1,01(d, J=7Hz, 3H); 1,24(s, 3H); 1,26(s, 3H); 1,33(s, 3H); 1,47(s, 3H); env.1,70(m, 3H) ;2,15(s, 3H); 2,26(s, 3H); 2,41(s, 3H); 7,00(s, 1H) delta ppm.

SM : 244($M^+$, 30), 229(85), 187(100), 173(58), 157(10).

e) On a procédé selon l'exemple 1b) (schéma I).

Dans la réaction d'halogénation on a utilisé les quantités et réactifs suivants : NBS (38,29 g), 1,2,3,4-tétrahydro-1,1,2,4,4,5,6,7-octaméthyl-naphtalène (préparé en d), 50,0 g), $CCl_4$ (400 ml). Après 45 min, on a procédé au traitement usuel pour obtenir 54,2 g de produit brut contenant le mélange de bromures benzyliques et environ 15% en poids de l'octaméthylnaphtalène de départ non-réagi.

Ce produit brut (54,2 g) a été mélangé avec la N-méthylpyrrolidone (300 ml) et l'eau (45 ml). La réaction d'hydrolyse a fourni 39,1 g de produit brut contenant les 5,6,7,8-tétrahydro-1,3,5,5,7,8,8-heptaméthyl-2-naphtalèneméthanol, 5,6,7,8-tétrahydro-3,4,5,5,6,8,8-heptaméthyl-2-naphtalèneméthanol, et 5,6,7,8-tétrahydro-2,3,5,5,7,8,8-heptaméthyl-1-naphtalèneméthanol, ainsi qu'environ 15% en poids de l'octaméthylnaphtalène de départ non-réagi.

Ce produit brut (39,1 g, env. 83% en poids d'alcools) a été utilisé dans la réaction d'oxydation, avec 44,9 g de PCC et 300 ml de $CH_2Cl_2$. Après la filtration on a obtenu deux fractions, une contenant 6,02 g de l'octa-méthylnaphtalène de départ, et l'autre contenant 5,00 g d'un mélange des trois aldéhydes. La chromatographie en phase gazeuse a permis de séparer le 5,6,7,8-tétrahydro-1,3,5,5,7,8,8-heptaméthyl-2-naphtalène-carbaldéhyde pur, et un échantillon de 5,6,7,8-tétrahydro-3,4,5,5,6,8,8-heptaméthyl-2-naphtalènecarbaldéhyde contenant encore 10% en poids du composé précédent.

IR (mélange, $CDCl_3$) : 2950, 2920, 1680, 1580, 1455, 1360 $cm^{-1}$

5,6,7,8-tétrahydro-1,3,5,5,7,8,8-heptaméthyl-2-naphtalènecarbaldéhyde

RMN ($^1H$, 360MHz) : 1,01(d,J = 7Hz, 3H); 1,25(s,3H); 1,30(s, 3H); 1,35(s, 3H); 1,46(s, 3H); env.1,40(m, 1H); 1,64(t, J = 13Hz, 1H); 1,79(m, 1H); 2,47(s,3H); 1,32(s, 3H); 7,05(s, 1H); 10,60(s, 1H) delta ppm

SM : 258($M^+$, 27), 243(38), 201(41), 187(55), 173(100), 159(32), 143(43), 128(25), 115(25), 105(24), 91(35), 77(21), 57(23), 43(24).

5,6,7,8-tétrahydro-3,4,5,5,6,8,8-heptaméthyl-2-naphtalènecarbaldéhyde

RMN ($^1H$, 360MHz) : 1,02(d, J = 7Hz, 3H); 1,28(s, 3H); 1,32(s, 3H); 1,35(s,3H); 1,47(s, 3H); env.1,40-(m, 1H); 1,64(t, J = 13Hz, 1H); 1,82(m, 1H); 2,44(s, 3H); 2,54(s, 3H); 7,66(s, 1H); 10,27(s, 1H) delta ppm.

SM : 258($M^+$, 27), 243(38), 201(41), 187(55), 173(100), 159(32), 143(43), 128(25), 115(25), 105(24), 91(35), 77(21), 57(23), 43(24).

Le mélange de ces deux composés possédait une note bien musquée, animale, brûlée.

Exemple 4

Préparation de trans-5,6,7,8-tétrahydro-3,5,5,6,7,8,8-heptaméthyl-2-naphtalènecarbaldéhyde

a) Préparation de 2,2,4,5-tétraméthyl-5-hexén-3-one

Dans un ballon à sulfuration de 1 l sous $N_2$, muni d'une agitation mécanique, d'un thermomètre et d'un réfrigérant, on a additionné successivement à 20° de l'isoprène récemment distillé (30,0 g), du PrMgBr [(Pr = propyle, 213 ml à 1,88 N); préparé à partir de PrBr (54,2 g), Mg (12,7 g) et $Et_2O$ (200 ml)], du $Cp_2TiCl_2$ (Fluka, 1 g). Après 15 h à 20°, la solution a été transvasée (par canule via ampoule à introduction) dans un deuxième ballon de 1,5 l (équipé comme le premier), refroidi à -10° et contenant du chlorure de pivaloyle (53,0 g) en solution dans $Et_2O$ (100 ml). On a agité 1 h, versé sur $NH_4Cl$ sat. extrait ($Et_2O$) et lavé les phases organiques avec NaOH 5%, $H_2O$ et NaCl saturé, puis séché ($Na_2SO_4$), filtré et évaporé sous vide dans un appareil de distillation. On a isolé 49,2 g d'un liquide jaune. La cétone désirée a été distillée sur un simple pont (50°/1,33x$10^3$ Pa). On a obtenu 39,2 g (rend. 61%) de 2,2,4,5-tétraméthyl-5-hexén-3-one (96% pur).

IR ($CDCl_3$) : 3050, 1700, 1640, 1470, 1360, 990 $cm^{-1}$

RMN ($^1H$, 60MHz) : 4,80(s, 2H); 3,71(q, J = 7Hz, 1H); 1,74(large, 3H); 1,17(d,J = 7Hz, 3H); 1,12(s, 9H) delta ppm.

SM : 154($M^+$, 3), 85(32), 69(14), 57(100), 41(34).

b) Préparation de 5-(3,4-diméthyl-1-phényl)-2,2,4,5-tétraméthyl-3-hexanone

Une solution de la cétone préparée en a) (37,2 g) dans le o-xylène (50 ml) a été additionnée goutte à goutte à une suspension de $AlCl_3$ (36,2 g) dans le o-xylène (380 ml), pendant 1 h, en maintenant la température à 0°. On a laissé remonter la température à 10° (env. 30 min), versé sur $H_2O$, extrait à l'éther et lavé avec $Na_2CO_3$, puis NaCl saturé. On a séché sur $Na_2SO_4$ et concentré par distillation sur un simple pont. On a obtenu 54,4 g (97% pur, rend. 88%) de la cétone désirée.

P. Eb. env. 120°/1,33x$10^2$ Pa

IR ($CDCl_3$) : 2950, 1690, 1470, 1360, 990 $cm^{-1}$

RMN ($^1H$, 60MHz) : 7,05(large, 3H); 3,27(q, J = 7Hz, 1H); 2,26(s, 3H); 2,22(s, 3H); 1,46(s, 3H); 1,37(s, 3H); 0,96(s, 9H); 0,95(d, J = 7Hz, 3H) delta ppm

SM : 260($M^+$, 1), 147(100), 131(8), 119(17), 91(10), 57(12), 41(10).

c) Préparation de 5-(3,4-diméthyl-1-phényl)-2,2,4,5-tétraméthyl-3-hexanol

Dans un ballon de 1 l, équipé d'une agitation mécanique, d'un thermomètre, d'un réfrigérant et maintenu sous azote, on a ajouté à une suspension de $LiAlH_4$ (3,80 g) dans l'$Et_2O$ (250 ml) une solution de la cétone préparée en b) (54,4 g) dans l'$Et_2O$ (50 ml). Après refroidissement à 10°, on a ajouté prudemment goutte à goutte 4 ml d'eau, puis 4 ml de NaOH 5% et 12 ml d'eau. L'alcool formé a été filtré, concentré et distillé (simple pont : 130-140°/2,0x$10^2$ Pa). On a obtenu 31,5 g du produit désiré (98% pur ; rend. 97% ; mélange de diastéreoisomères 94:6).

IR : 3600, 2980, 1840, 1370, 1010 $cm^{-1}$

RMN ($^1H$, 360MHz, + $D_2O$) : 7,18(s, 1H); 7,14(large d, J = 7,5Hz, 1H); 7,07(d,J = 7,5Hz, 1H); 3,09(d, J = 7,5Hz, 1H); 2,04(s, 3H); 2,02(s, 3H); 2,01(q,J = 7Hz, 1H); 1,46(s, 3H); 1,20(s, 3H); 1,05(d, J = 7Hz, 3H); 0,81(s,9H) delta ppm.

SM : 244(trace, $M^+$, 18), 187(7), 173(7), 147(100), 131(8), 119(17), 107(9), 91(10), 57(8), 41(13).

d) Préparation de trans-1,2,3,4-tétrahydro-1,1,2,3,4,4,6,7-octaméthylnaphtalène et cis-1,2,3,4-tétrahydro-1,1,2,3,4,4,6,7-octaméthylnaphtalène

L'alcool préparé selon c) (41,6 g) a été additionné sous agitation et refroidissement externe à un mélange d'acide méthanesulfonique (21,25 g) et $P_2O_5$ (8,5 g). On a maintenu la température à 40° pendant 4 h. Le mélange réactionnel a été refroidi, rendu plus fluide à l'aide de $CH_2Cl_2$ (10 ml) et

transvasé dans un bécher de 1 l contenant un mélange eau/glace. L'hydrocarbure formé a été extrait à l'éther, lavé avec NaOH 5%, $H_2O$, puis NaCl saturé, séché sur $Na_2SO_4$ et concentré. On a obtenu 37,3 g de produit brut contenant un mélange d'isomères trans-et cis- dans les proportions respectives de 3:1. La cristallisation dans l'éthanol, la distillation des eaux-mères (110°/1,33x$10^2$ Pa) et la cristallisation des fractions de distillation ont fourni 16,7 g de l'isomère transdésiré (98% pur, rend. 43%) et une huile contenant un mélange d'isomères transet cis- (19,8 g, environ 56% pur, trans/cis environ 40:60). Résidus : 0,52 g.

trans-1,2,3,4-tétrahydro-1,1,2,3,4,4,6,7-octaméthylnaphtalène

IR ($CHCl_3$) : 2990, 1500, 1450, 1400, 1370 $cm^{-1}$

RMN ($^1H$, 360MHz) : 7,12(s, 2H); 2,23(s, 6H); 1,58(m, 2H); 1,31(s, 6H); 1,09(s, 6H); 0,96(d, J=6Hz, 6H) delta ppm

RMN ($^{13}C$, 360MHz) : 143,1(s), 133,6(s), 128,2(d), 39,5(d), 37,5(s), 29,6(q), 25,7(q), 19,5(q), 13,9(q) delta ppm.

SM : 244($M^+$, 7), 229(24), 187(43), 173(100), 157(12), 145(23), 128(11), 91(8), 57(38), 41(9).

cis-1,2,3,4-tétrahydro-1,1,2,3,4,4,6,7-octaméthylnaphtalène

IR ($CHCl_3$) : 2990, 1500, 1450, 1400, 1370 $cm^{-1}$

RMN ($^1H$, 360MHz) : 7,08(s, 2H); 2,23(s, 6H); 1,88(large q, 2H); 1,26(s, 6H); 1,25(s, 6H); 0,95(d, J=7Hz, 6H) delta ppm

RMN ($^{13}C$, 360MHz) : 142,0(s), 133,6(s), 127,9(d), 41,4(d), 37,1(s), 33,7(q), 27,7(q), 19,4(q), 13,3(q) delta ppm.

SM : 244($M^+$, 7), 229(24), 187(43), 173(100), 157(12), 145(23), 128(11), 91(8), 57(38), 41(9).

e) A une solution de trans-1,2,3,4-tétrahydro-1,1,2,3,4,4,6,7-octaméthyl-naphtalène (obtenu en d), 16,0 g) dans le méthanol (700 ml) ont été ajoutées 16 portions de $Ce(NH_4)_2(NO_3)_6$ (16x16,0 g=256,0 g) dans le méthanol (16x100 ml), durant 8 h en maintenant la température à 50°. Environ 2/3 du méthanol ont été chassés au rotavapor, et le produit a été extrait avec éther de pétrole 30-50°/NaCl saturé. Le produit brut obtenu (19,1 g) contenait un nouveau produit plus lourd non-identifié. Après cristallisation dans l'éthanol, traitement acide des eaux-mères, et nouvelles cristallisations, on a isolé 12,1 g de trans-5,6,7,8-tétrahydro-3,5,5,6,7,8,8-heptaméthyl-2-naphtalènecarbaldéhyde (98,5% pur, rend. 80%) désiré.

IR ($CDCl_3$) : 2960, 1680, 1600, 1450, 1360, 1205 $cm^{-1}$

RMN ($^1H$, 360MHz) : 10,19(s, 1H); 7,80(s, 1H); 7,21(s, 1H); 2,61(s, 3H); 1,59(m, 2H); 1,35(s, 3H); 1,33(s, 3H); 1,12(s, 6H) ;0,99(d, J=6Hz,6H) delta ppm.

SM : 258($M^+$, 24), 243(58), 201(30), 187(100), 173(40), 159(34), 141(18), 131(23), 115(15), 57(12), 43-(47).

Les propriétés odorantes de ce composé ont été décrites dans l'introduction de cette description.

## Exemple 5

### Préparation de cis-5,6,7,8-tétrahydro-3,5,5,6,7,8,8-heptaméthyl-2-naphtalènecarbaldéhyde

Ce produit, diastéreoisomère du composé préparé dans l'exemple 4, a été obtenu par oxydation d'un mélange contenant les deux hydrocarbures isomères préparés dans l'exemple 4d), suivie de chromatographie en phase préparative.

IR ($CDCl_3$) : 2960, 1680, 1600, 1450, 1360, 1205 $cm^{-1}$

RMN ($^1H$, 360MHz) : 10,20(s, 1H); 7,76(s, 1H); 71,8(s, 1H); 2,62(s, 3H); 1,92(m, 2H); 1,32(s, 3H); 1,31(s, 3H); 1,28(2s, 6H) ;0,95(d, J=7Hz, 6H) delta ppm.

RMN ($^{13}C$) : 192,7(d), 151,3(s), 143,0(s), 137,2(s), 132,2(s), 131,3(d), 130,2(d), 41,1(d), 41,1(d), 38,0(s), 37,4(s), 33,7(q), 33,4(q), 27,6(q), 27,4(q), 19,2(q), 13,2(q), 13,2(q) delta ppm

SM : 258($M^+$, 24), 243(58), 201(30), 187(100), 173(40), 159(34), 141(18), 131(23), 115(15), 57(12), 43-(47).

Ce composé possédait une note musquée, terreuse, légèrement animale.

## Exemple 6

### Préparation de trans-(5,6,7,8-tétrahydro-3,5,5,6,7,8,8-heptaméthyl-2-naphtyl)-1-éthanone

A une solution de l'aldéhyde préparé dans l'exemple 4(7,0 g) dans le THF (40 ml) on a ajouté une solution MeMgCl/THF (9,7 ml, 3,54 N) en maintenant la température de la réaction à 20° (bain de glace).

Le mélange réactionnel a été hydrolysé avec $NH_4Cl$, extrait à l'éther et lavé avec $H_2O$ et NaCl saturé. On a séché sur $Na_2SO_4$ et concentré. On a obtenu 6,9 g de produit brut. Une solution de 6,26 g de ce produit dans le $CH_2Cl_2$ (60 ml) a été ajoutée goutte à goutte à une solution de PCC (7,86 g) dans le $CH_2Cl_2$ (20 ml). On a agité le mélange pendant 2 h, en maintenant la température à 20°, filtré sur $SiO_2$ sous légère pression d'azote, concentré (5,86 g) et cristallisé dans l'éthanol. Les eaux-mères ont été chromatographiées ($SiO_2$ ; $CH_2Cl_2$) et cristallisées. On a obtenu au total 4,3 g de trans-(5,6,7,8-tétrahydro-3,5,5,6,7,8,8-heptaméthyl-2-naphtyl)-1-éthanone (95% pur, rend. 62%).

IR ($CDCl_3$) : 2960, 1670, 1440, 1350, 1220 $cm^{-1}$

RMN ($^1H$, 360MHz, $CDCl_3$) : 7,73(s, 1H); 7,20(s, 1H); 2,45(s, 3H); 2,40(s, 3H); 1,58(m, 2H); 1,30(s, 6H); 1,06(s, 6H) ;0,91(d, J = 6,5Hz, 6H) delta ppm.

SM : 272($M^+$, 7), 257(22), 215(14), 201(40), 173(23), 159(16), 141(16), 128(17), 115(12), 57(13), 41(100).

Les propriétés odorantes de ce composé ont été décrites en détail dans l'introduction de cette description.

Exemple 7

Préparation de 1,2,6,7,8,8a-hexahydro-3,6,6,8a-tétraméthyl-4-acenaphtylènecarbaldéhyde

a) Préparation de 2-acétyl-3-(2-méthyl-1-phényl)propanoate d'éthyle

Dans un ballon à sulfuration de 2,5 l équipé d'une agitation mécanique, d'un réfrigérant, d'un thermomètre et d'une entrée d'azote, on a mélangé de l'alphachloro-o-xylène (Fluka, 140,6 g), de l'acétoacétate d'éthyle (130 g), du carbonate de potassium en poudre fine (414 g) et 800 ml de toluène. On a chauffé 20 h à 100°. On a refroidi, additionné $H_2O$ (500 ml). La phase organique a été lavée à l'eau, au NaCl saturé, puis séchée sur $Na_2SO_4$ et évaporée. On a obtenu 269,6 g d'une huile brune qui a été distillée (120-125°/5,65 Pa) pour fournir 163 g de 2-acétyl-3-(2-méthyl-1-phényl)propanoate d'éthyle (98% pur, rend. 70%).

b) Préparation de 4-(2-méthyl-1-phényl)-2-butanone

Dans un autoclave de 1 l on a introduit 161,5 g du 2-acétyl-3-(2-méthyl-1-phényl)propanoate d'éthyle obtenu en a), 16,4 g de NaCl, 150 ml de DMSO et 25 ml de $H_2O$. On a chauffé à 160° pendant 7 h. Le mélange réactionnel refroidi a été extrait à l'éther de pétrole 30-50°, lavé 5 fois avec une solution saturée de NaCl, séché sur $Na_2SO_4$, filtré et évaporé. La distillation (13,3 Pa) a fourni 101 g de la butanone désirée (98% pur, rend. 91%).

P. Eb. 65-70°/13,3 Pa

IR : 2925, 1705, 1490, 1350, 1160 $cm^{-1}$

RMN ($^1H$, 60MHz) : 2,10(s, 3H); 2,28(s, 3H); 2,65-3,00(m, 4H); 7,07(s, 4H) delta ppm

SM : 162($M^+$,2), 144(100), 129(50), 119(52), 105(83), 91(45), 77(28), 65(18), 43(58).

c) Préparation de 7-(2-méthyl-1-phényl)-2,5-diméthylhept-3-yne-2,5-diol

Dans un ballon de 1 l, on a ajouté du 2-méthyl-3-butyn-2-ol (47 g) à une solution de EtMgBr (1,12 mole de Mg et 1,12 mole de bromure d'éthyle dans 200 ml d'éther anhydre à reflux) pendant 30 min, en maintenant la température à 0-5°. Le mélange hétérogène a été chauffé pendant 30 min à 20°, sous agitation, ensuite à reflux pendant 1 h. On a ajouté 69,7 g de 4-(2-méthyl-1-phényl)-2-butanone préparé selon b), et chauffé à reflux pendant 1 h. Le mélange de la réaction, qui est devenu homogène, a été hydrolysé avec un mélange d'une solution aqueuse saturée de $NH_4Cl$ et glace, extrait à l'éther, lavé avec NaCl saturé, séché et évaporé. On a obtenu 107,9 g d'huile jaune (rend. env. 100%) constituée par le diol désiré.

IR : 3350 $cm^{-1}$

RMN ($^1H$, 60MHz) : 1,50(s, 9H); 1,70-2,03(m, 2H); 2,30(s, 3H); 2,63-2,94(m, 2H); 2,94(large s, 2H); 7,13(s, 4H) delta ppm.

d) Préparation de 7-(2-méthyl-1-phényl)-2,5-diméthylheptane-2,5-diol

60 g du diol préparé en c) ont été hydrogénés dans un autoclave, à 70° et 50 atmosphères de $H_2$, en présence d'environ 3,0 g de Ni de Raney dans du MeOH (80 ml). Après 4 jours dans ces conditions, on a filtré la suspension et évaporé le filtrat, pour obtenir 60 g de 7-(2-méthyl-1-phényl)-2,5-diméthylheptane-2,5-diol (rend. env. 100%).

IR : 3350, 2930, 1455, 1370 $cm^{-1}$

RMN ($^1H$, 60MHz) : 1,22(2s, 9H); 1,60(s, 4H); 1,50-1,90(m, 2H); 2,22(large s, 2H, échange avec $D_2O$); 2,30(s, 3H); 2,45-2,85(m, 2H); 7,12(s,4H) delta ppm

e) Préparation de 1,2,2a,3,4,5-hexahydro-2a,5,5,8-tétraméthylacénaphtène

Une solution agitée et refroidie (4°) du diol préparé en d) (12,5 g) dans du 1,2-dichloroéthane (150 ml) a été traitée goutte à goutte avec du $TiCl_4$ (16,5 ml). On a agité pendant 30 min, puis introduit goutte

à goutte une solution aqueuse saturée de NaCl (50 ml), la température montant à 30°. On a lavé avec une solution aqueuse saturée de $NaHCO_3$, puis avec une solution aqueuse saturée de NaCl, séché sur $Na_2SO_4$, évaporé et distillé (130°/2,66 Pa). On a obtenu 7,76 g du produit désiré (rend. 77%).

IR : 2920, 1485, 1445, 1360 $cm^{-1}$

RMN ($^1H$, 360MHz) : 1,12(2s, 6H); 1,38(s, 3H); 1,60-1,85(m, 4H); 2,02(m, 2H); 2,22(s, 3H); 2,68(m, 1H); 2,97(m, 1H); 6,94(d, J=8Hz, 1H); 7,01(d, J=8Hz, 1H) delta ppm.

SM : 214($M^+$, 15), 199(100), 157(18), 143(14).

f) Le précurseur 1,2,2a,3,4,5-hexahydro-2a,5,5,8-tétraméthylacénaphtène obtenu selon e) a été traité de façon semblable à celle décrite dans l'exemple 1c), avec $TiCl_4$ et $Cl_2CHOCH_3$. On a utilisé 4,71 g de précurseur dans la réaction d'acylation et obtenu 3,14 g de 1,2,6,7,8,8a-hexahydro-3,6,6,8a-tétraméthyl-4-acénaphthylènecarbaldéhyde (rend. 59%).

IR ($CDCl_3$) : 2950, 2855, 1680, 1590, 1450 $cm^{-1}$

RMN ($^1H$, 60MHz) : 1,15(s, 6H); 1,41(s, 3H); 1,65-2,30(m, 6H); 2,52(s, 3H); 2,70-3,15(m, 2H); 7,60(s, 1H); 10,23(s, 1H) delta ppm.

SM :242($M^+$, 18), 227(100), 199(20), 165(10), 157(36), 143(25), 128(17), 115(14), 92(12), 69(11).

Ce composé possédait une jolie note musquée avec un côté musc Ambrette assez faible.

Exemple 8

Préparation de (1,2,6,7,8,8a-hexahydro-3,6,6,8a-tétraméthyl-4-acénaphtylényl)-1-éthanone

Une solution de l'hydrocarbure préparé dans l'exemple 7e)(1,28 g) dans le 1,2-dichloroéthane a été ajoutée goutte à goutte à une suspension de $AlCl_3$ (960 mg) dans le 1,2-dichloroéthane (10 ml). On a ensuite ajouté à la suspension orange 518 g de chlorure d'acétyle. Après 30 min, de l'eau a été ajoutée et le produit a été extrait à l'éther. La phase organique a été lavée avec une solution aqueuse saturée de $NaHCO_3$, ensuite avec une solution aqueuse saturée de NaCl, séchée sur $Na_2SO_4$, évaporée et purifiée par chromatographie sur colonne ($SiO_2$, cyclohexane/acétate d'éthyle, 95:5). On a obtenu 0,80 g de la cétone désirée (rend. 52%).

IR ($CHCl_3$) : 2920, 2850, 1675, 1445, 1345, 1290, 1245 $cm^{-1}$

RMN ($^1H$, 360MHz) : 1,13(s, 3H); 1,15(s, 3H); 1,40(s, 3H); 1,58-1,85(m, 4H); 1,98-2,09(m, 2H); 2,37(s, 3H); 2,56(s, 3H); 2,74(m, 1H); 2,99(m, 1H); 7,43(s, 1H) delta ppm.

SM : 256($M^+$, 11), 241(87), 199(20), 153(10), 43(100).

Ce composé possédait une note musquée avec un côté caractéristique des composés musqués nitro-aromatiques.

Exemple 9

Préparation d'un mélange de 2,3,3a,4,5,9b-hexahydro-5,5,8,9b-tétraméthyl-1H-benz[e]indène-7-carbaldéhyde et 2,3,3a,4,5,9b-hexahydro-5,5,7,9b-tetraméthyl-1H-benz[e]indène-8-carbaldéhyde

a) Préparation de 1-(2-méthyl-2-propényl)-2-oxo-1-cyclopentanecarboxylate de méthyle

Un mélange de 1-cyclopentanone-2-carboxylate de méthyle (Fluka, 106,5 g), chlorure de méthallyle (88 ml), $K_2CO_3$ (207 g) et acétone (500 ml) a été chauffé à reflux pendant 2 h. On a rajouté du chlorure de méthallyle (44 ml) et chauffé à reflux pendant 20 h. La masse blanche de la réaction a été dissoute dans de l'eau et le produit extrait à l'éther. Le lavage (NaOH aq. 3%) et le traitement usuel ont fourni 148 g d'une huile qui a été soumise à une distillation fractionnée pour donner 113 g du cétoester désiré (rend. 77%, P. Eb. 105-107°/5,32x$10^2$ Pa).

IR : 2950, 1750, 1720, 1430, 1210 $cm^{-1}$

RMN ($^1H$, 60MHz) : 1,64(s, 3H); 1,70-3,00(m, 8H); 3,68(s, 3H); 4,73(large s, 1H); 4,85(large s, 1H) delta ppm.

SM : 178($M^+$, 16), 168(16), 140(45), 136(30), 121(36), 109(100), 93(35), 79(60), 67(36), 55(41), 39-(33).

b) Préparation de 2-(2-méthyl-2-propényl)-1-cyclopentanone

Un mélange du cétoester préparé en a) (78,4 g), HMPA (hexaméthylphosphoreamidure, 250 ml) et LiCl (34 g) a été chauffé à 73° pendant 36 h. L'extraction (3 fois, éther/eau) du produit de la réaction a fourni 70,3 g d'une huile brune qui a été soumise à une distillation fractionnée pour fournir 34,7 g de la cétone susmentionnée (rend. 62%, P. Eb. 63-75°/5,32x$10^2$ Pa).

RMN ($^1H$, 60MHz) : 1,71(s, 3H); env.1,50-2,70(m, 9H); 4,68(large s, 2H) delta ppm

SM : 138($M^+$, 45), 123(12), 110(38), 95(37), 82(100), 67(77), 55(38), 41(35).

c) Préparation d'un mélange de 2-[2-méthyl-2-(4-méthyl-1-phényl)propyl]-1-cyclopentanone et 2-[2-méthyl-2-(3-méthyl-1-phényl)propyl]-1-cyclopentanone

10,35 g de la cétone préparée en b) ont été ajoutés goutte à goutte à une suspension de $AlCl_3$ (15 g) dans le toluène (138 g) à -20°. On a laissé la température remonter à 10°, agité pendant 30 min, hydrolysé à l'eau et extrait à l'éther. Une partie (6 g) de l'huile jaune obtenue (16,56 g, rend. 95%) a été distillée au four à boules (130°/1,33 Pa) pour fournir 5,2 g d'un mélange 3:1 des cétones susmentionnées (rend. 83%).

IR : 2950, 1735, 1510, 1450, 1150 $cm^{-1}$

RMN ($^1H$, 60MHz, pics caractéristiques) : 1,28(s, 6H); 2,30(large s, 3H); 6,90-7,30(m, 4H) delta ppm.

SM : 133($M^+$, 100), 105(25), 91(10), 41(12).

d) Préparation d'un mélange de 1-méthyl-2-[2-méthyl-2-(4-méthyl-1-phényl) propyl]-1-cyclopentanol et 1-méthyl-2-[2-méthyl-2-(3-méthyl-1-phényl) propyl]-1-cyclopentanol

Une solution de MeMgI (préparée à partir de 3,4 ml de MeI et 1,2 g de Mg) dans l'éther (100 ml) a été traitée à 20° avec une solution du mélange brut de cétones préparé en c) (10,44 g) dans l'éther (10 ml). Une fois l'addition de cétones terminée (env. 10 min) on a hydrolysé et extrait le mélange d'alcools qui a été utilisé sans purification dans l'étape suivante.

e) Préparation d'un mélange de 1-[1,1-diméthyl-2-(2-méthyl-1-cyclopentén-1-yl)éthy]-4-méthylbenzène et 1-[1,1-diméthyl-2-(2-méthyl-1-cyclopentén-1-yl)éthyl]-3-méthylbenzène

Une solution du mélange d'alcools brut préparé en d) (9,84 g), d'éther de pétrole 30-50° (150 ml) et de $H_2SO_4$ 98% (0,75 ml) a été agitée pendant 1 h, en maintenant la température à 20°. Le mélange de la réaction a été extrait et distillé au four à boules (130°/5,65 Pa) pour fournir 6,89 g d'un mélange des oléfines susmentionnées.

RMN ($^1H$, 60MHz, pics caractéristiques) : 1,25(s, 6H); 1,54(large s, 3H); 2,29(large s, 3H); 6,88-7,30-(m, 4H) delta ppm.

f) Préparation d'un mélange de 1,2,3,3a,4,5-hexahydro-1a,5,5,8-tétraméthylacénaphtylène et 1,2,3,3a,4,5-hexahydro-1a,5,5,7-tétraméthylacénaphtylène

Une solution du mélange d'oléfines préparée en e) (5,5 g), d'éther de pétrole 30-50° (120 ml) et de $H_2SO_4$ 98% (0,5 ml) a été chauffée à reflux (50°) pendant 5 h. Extraction et distillation au four à boules (140°/13,3 Pa) ont permis d'obtenir un mélange des hydrocarbures susmentionnés.

RMN ($^1H$, 60MHz, pics caractéristiques) : 1,20-1,30(4s, 9H); 2,30(s, 3H); 6,80-7,30(m, 3H) delta ppm.

g) La réaction d'acylation des hydrocarbures préparés en f) a été effectuée selon l'exemple 1c) en utilisant du $TiCl_4$ (1,86 ml) dans le chlorure de méthylène (30 ml) et du $Cl_2CHOCH_3$ (0,8 ml) dans le chlorure de méthylène (5 ml). On a obtenu 1,05 g d'un mélange des isomères 2,3,3a,4,5,9b-hexahydro-5,5,8,9b-tétraméthyl-1H-benz[e]indène-7-carbaldéhyde et 2,3,3a,4,5,9b-hexahydro-5,6,7,9b-tétraméthyl-1H-benz[e]indène-8-carbaldéhyde dans les proportions respectives de 3:1 (rend. 45%).

IR : 2940, 2850, 1680, 1600, 1540, 1440, 1205 $cm^{-1}$

RMN ($^1H$, 60MHz) : 1,22-1,42(4s, 9H); 1,40-2,30(m, 9H); 2,62(s, 3H); 7,10(7,17*)(s, 1H); 7,70(7,64*)(s, 1H); 10,14(s, 1H) delta ppm.

SM : isomère majoritaire - 256($M^+$, 29), 241(100), 199(11), 185(45), 171(58),157(45), 143(28), 128-(22), 69(24).

SM : isomère minoritaire - 256($M^+$, 57), 241(100), 227(37), 214(23), 199(27), 185(57), 171(100), 157-(57), 143(38), 128(35), 115(23), 69(28), 55(26), 41(28).

Ce composé possédait une note musquée, fleurie.

## Exemple 10

Préparation d'un mélange de 1-(2,3,3a,4,5,9b-hexahydro-5,5,8,9b-tétraméthyl-1H-benz[e]indène-7-yl)-1-éthanone et 1-(2,3,3a,4,5,9b-hexahydro-5,5,7,9b-tétraméthyl-1H-benz[e]-indén-8-yl)-1-éthanone

Ce mélange de cétones a été obtenu en partant du mélange d'hydrocarbures préparé dans l'exemple 9f) et selon un procédé analogue à celui décrit dans l'exemple 8. On a obtenu 0,45 g d'un mélange des cétones isomériques susmentionnées dans les proportions de 3:1.

* pics correspondants à l'isomère minoritaire

RMN ($^1H$, 60MHz) : 1,19-1,34(5s, 9H); 1,40-2,30(m, 9H); 2,49 et 2,53(2s + 2 épaules, 6H); 7,03(7,06*)(s, 1H); 7,60(7,53*)(s, 1H) delta ppm.

SM : 270($M^+$, 13) 255(43), 213(8), 199(8), 185(8), 171(7), 153(8), 141(8), 128(10), 115(10), 91(8), 43-(100).

Ce composé possédait une note musquée, fruitée.

Exemple 11

Composition de base de type florale pour détergent en poudre

Une composition de base de type florale pour un détergent en poudre a été préparée avec les ingrédients suivants :

| Ingrédients | Parties en poids |
|---|---|
| Acétate de diméthylbenzylcarbinyle | 100 |
| Aldéhyde hexylcinnamique | 2500 |
| Ambrox (marque enregistrée) DL 1) à 10%* | 50 |
| Acétate d'isononyle | 250 |
| Propionate de verdyle | 800 |
| Coumarine | 100 |
| Aldéhyde cyclamen | 350 |
| Acétate de p-tert-butylcyclohexanol | 1100 |
| Fleuramone (marque enregistrée) 2) | 200 |
| Isoraldeine (marque enregistrée) 70 3) | 300 |
| Lilial (marque enregistrée) 4) | 800 |
| Alcool phényléthylique | 800 |
| Salicylate de benzyle | 400 |
| Tétrahydrolinalol | 400 |
| Verdox (marque enregistrée) 5) | 250 |
| Vertofix coeur (marque enregistrée) 6) | 500 |
| Total | 9000 |

* dans le dipropylèneglycol
1) tétraméthyl perhydronaphtofuranne; origine : Firmenich SA, Genève
2) 2-heptyl-1-cyclopentanone ; origine : International Flavors & Fragrances Inc.
3) iso-méthylionone ; origine : L. Givaudan SA, Genève
4) 3-(4-tert-butyl-1-phényl)-2-méthylpropanal ; origine : L. Givaudan SA, Genève
5) acétate de 2-tert-butyl-1-cyclohexyle ; origine : International Flavors & Fragrances Inc.
6) origine : International Flavors & Fragrances Inc.

Avec cette base on a préparé quatre compositions parfumantes avec les ingrédients suivants (parties en poids) :

* pics correspondant à l'isomère minoritaire

| | A | B | C | D |
|---|---|---|---|---|
| Base | 9000 | 9000 | 9000 | 9000 |
| trans-5,6,7,8-tétrahydro-3,5,5,6,7,8,8-heptaméthyl-2-naphtalènecarbaldéhyde | 500 | 250 | --- | -- |
| trans-(5,6,7,8-tétrahydro-3,5,5,6,7,8,8-heptaméthyl-2-naphtyl)-1-éthanone | --- | --- | 1000 | --- |
| Tonalid (marque enregistrée)* | --- | --- | --- | 1000 |
| Dipropylèneglycol | 500 | 750 | --- | --- |
| Total | 10000 | 10000 | 10000 | 10000 |

* 7-acétyl-1,1,3,4,4,6-hexaméthyltétraline ; origine : Polak's Frutal Works

Ces diverses compositions ont été évaluées par un panel d'experts parfumeurs et il est apparu que la composition A développait une puissante note musquée-animale surdosée. La composition B, dont la concentration en trans-5,6,7,8-tétrahydro-3,5,5,6,7,8,8-heptaméthyl-2-naphtalènecarbaldéhyde était la moitié de celle de la composition A, était plus équilibrée que cette dernière, mais toujours très musquée-animale. En comparant cette composition B avec la composition D, on s'est aperçu que la première possédait un caractère très différent de la deuxième, et que sa note ambrée-animale était très perceptible. Quant à la composition C, elle se rapprochait de la composition D, de par son effet musqué plus floral, plus doux que celui des compositions A et B. Le caractère de la composition C était toutefois différent de celui de la composition D.

Ces compositions parfumantes ont été ajoutées à un détergent en poudre que l'on a ensuite utilisé pour le lavage de linge. Ce dernier a été séché à l'air libre pendant 24 heures. Les différences citées ci-dessus sont alors devenues encore plus apparentes. Par ailleurs, la puissance de la composition B par rapport à la composition D était encore plus accentuée, et la première composition possédait une meilleure substantivité.

Exemple 12

Composition de base pour une lotion masculine.

Une composition parfumante de base pour une lotion masculine a été préparée avec les ingrédients suivants :

| Ingrédients | Parties en poids |
| --- | --- |
| Acétate de géranyle à 10%* | 150 |
| Acétate de lynalyle | 200 |
| Undécalactone gamma à 1%* | 100 |
| Bergamote | 1500 |
| Essence de citron de Sicile | 150 |
| Coumarine | 125 |
| Dihydromyrcenol [1)] | 200 |
| Florol (marque enregistrée) [2)] | 50 |
| Indol purifié à 10%* | 75 |
| Iso E Super [3)] | 2000 |
| Isobutylquinoléine à 10%* [4)] | 25 |
| Lyral (marque enregistrée) [5)] | 600 |
| Essence de mandarine | 700 |
| Mousse cristal à 10%* | 350 |
| Muscade | 100 |
| Hédione (marque enregistrée) [6)] | 350 |

| | |
|---|---|
| Octine carbonate de méthyle à 10%* | 850 |
| Patchouli | 200 |
| Salicylate d'amyle | 200 |
| Sandela (marque enregistrée) 7) | 350 |
| Vertofix coeur (marque enregistrée) 8) | 1150 |
| Bêta-ionone à 10%* | 100 |
| Cyclal C à 10%* 9) | 75 |
| Timberol (marque enregistrée) 10) | 150 |
| Total | 9750 |

* dans le dipropylèneglycol

1) origine : International Flavors & Fragrances Inc.

2) 4-méthyl-2-méthylpropyl-4-2H-pyranol ; origine : Firmenich SA, Genève

3) 2-acétyl-1,2,3,4,5,6,7,8-octahydro-2,3,8,8-tétraméthylnaphtalène ; origine : International Flavors & Fragrances Inc.

4) 6-(1-méthylpropylquinoléine) ; origine International Flavors & Fragrances Inc.

5) 4-(4-hydroxy-4-méthylpentyl)-3-cyclohexène-1-carboxaldéhyde ; origine : International Flavors & Fragrances Inc.

6) dihydrojasmonate de méthyle ; origine : Firmenich SA, Genève

7) 3-(isocamphyl-5-)-cyclohexan-1-ol ; origine : L. Givaudan S.A., Genève

8) voir exemple 11

9) 3,5-diméthyl-3-cyclohexén-1-carbaldéhyde ; origine : L. Givaudan S.A., Genève

10) 1-(2,2,6-triméthylcyclohexyl)-3-hexanol ; origine : Dragoco A.G., RFA

A cette composition de base on a ajouté 250 parties en poids de trans-5,6,7,8-tétrahydro-3,5,5,6,7,8,8-heptaméthyl-2-naphtalènecarbaldéhyde. On a ainsi obtenu une nouvelle composition dont la note s'est trouvée profondémment changée. Outre un effet ambré-animal très évident au départ, le mélange a pris beaucoup plus de volume et de puissance, l'effet musqué étant alors nettement perceptible. La lotion avait aussi pris une connotation plus masculine. Ces effets étaient encore plus évidents lorsque les notes de fond ont été senties sur la mouillette, 24 h plus tard.

## Revendications

**1.** Composé de formule

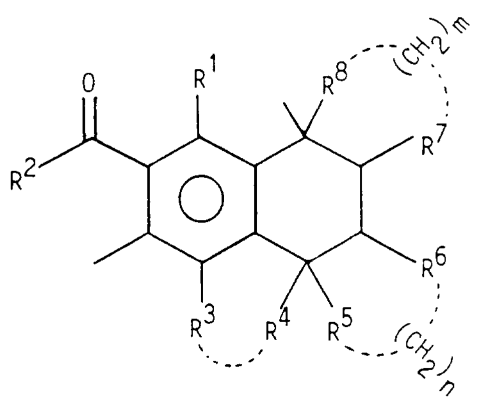

(I)

dans laquelle

a) les indices m et n sont identiques et définissent chacun un nombre entier de valeur 0, les symboles $R^1$ et $R^2$ sont identiques et représentent chacun un atome d'hydrogène, ou sont différents et représentent chacun un atome d'hydrogène ou un radical méthyle, les symboles $R^5$ et $R^8$ représentent chacun un radical méthyle, les symboles $R^6$ et $R^7$, identiques ou différents, représentent chacun un atome d'hydrogène ou un radical méthyle, et soit le symbole $R^4$ représente un radical méthyle et le symbole $R^3$ un atome d'hydrogène ou un radical méthyle, soit les symboles $R^3$ et $R^4$ représentent chacun un radical méthylène faisant partie d'un cycle tel qu'indiqué par la ligne pointillée, les combinaisons suivantes étant cependant exclues :

1. $R^1 = R^2 = R^3 = R^6 = R^7 = H$, ou
2. $R^1 = R^2 = R^3 = H$ et $R^6$ ou $R^7 = CH_3$, ou
3. $R^2 = CH_3$ et $R^3 = R^6 = R^7 = H$ ou
4. $R^2 = CH_3$, $R_1 = R^3 = H$ et $R^6$ ou $R^7 = CH_3$, ou
5. $R^1 = R^3 = CH_3$, ou
6. $R^3 = R^4 = CH_2$ et $R^6$ ou $R^7 = CH_3$ ;

ou dans laquelle

b) les indices m et n sont différents et définissent chacun un nombre entier de valeur 0 ou 1, le symbole $R^2$ représente un atome d'hydrogène ou un radical méthyle, les symboles $R^1$ et $R^3$ représentent chacun un atome d'hydrogène, le symbole $R^4$ représente un radical méthyle, et soit les symboles $R^5$ et $R^6$ sont identiques (n = 1) et représentent chacun un radical méthylène faisant partie d'un cycle tel qu'indiqué par la ligne pointillée, le symbole $R^7$ représentant un atome d'hydrogène et le symbole $R^8$ un radical méthyle, soit le symbole $R^5$ représente un radical méthyle et le symbole $R^6$ un atome d'hydrogène, les symboles $R^7$ et $R^8$ étant alors identiques (m = 1) et représentant chacun un radical méthylène faisant partie d'un cycle tel qu'indiqué par la ligne pointillée ;

ou tout mélange de deux ou plusieurs isomères structurels de formule (I).

**2.** A titre de composé de formule (I) selon la revendication 1, un composé de formule

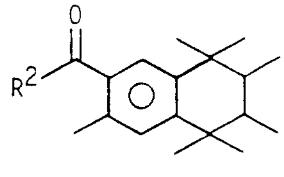

(II)

dans laquelle le symbole $R^2$ représente un atome d'hydrogène ou un radical méthyle.

**3.** A titre de composé de formule (I) selon la revendication 1, l'un des composés suivants :

a) 5,6,7,8-tétrahydro-1,3,5,5,8,8-hexaméthyl-2-naphtalènecarbaldéhyde

b) 5,6,7,8-tétrahydro-3,4,5,5,8,8-hexaméthyl-2-naphtalènecarbaldéhyde

c) 5,6,7,8-tétrahydro-1,3,5,5,6,8,8-heptaméthyl-2-naphtalènecarbaldéhyde
d) 5,6,7,8-tétrahydro-3,4,5,5,7,8,8-heptaméthyl-2-naphtalènecarbaldéhyde
e) 5,6,7,8-tétrahydro-1,3,5,5,7,8,8-heptaméthyl-2-naphtalènecarbaldéhyde
f) 5,6,7,8-tétrahydro-3,4,5,5,6,8,8-heptaméthyl-2-naphtalènecarbaldéhyde
g) 1,2,6,7,8,8a-hexahydro-3,6,6,8a-tétraméthyl-4-acénaphtylènecarbaldéhyde
h) (1,2,6,7,8,8a-hexahydro-3,6,6,8a-tétraméthyl-4-acénaphtylényi)-1-éthanone
ou tout mélange des composés a) et b), ou c) et d), ou e) et f).

4. A titre de composé selon la revendication 1, un mélange des composés i) et j) ou k) et l) suivants :
i) 2,3,3a,4,5,9b-hexahydro-5,5,8,9b-tétraméthyl-1H-benz[e]indène-7-carbaldéhyde
j) 2,3,3a,4,5,9b-hexahydro-5,5,7,9b-tétraméthyl-1H-benz[e]indène-8-carbaldéhyde
k) 1-(2,3,3a,4,5,9b-hexahydro-5,5,8,9b-tétraméthyl-1H-benz[e]indén-7-yl)-1-éthanone
l) 1-(2,3,3a,4,5,9b-hexahydro-5,5,7,9b-tétraméthyl-1H-benz[e]indén-8-yl)-1-éthanone.

5. A titre de composé selon la revendication 2, l'un des composés suivants :
m) trans-5,6,7,8-tétrahydro-3,5,5,6,7,8,8-heptaméthyl-2-naphtalènecarbaldéhyde
n) cis-5,6,7,8-tétrahydro-3,5,5,6,7,8,8-heptaméthyl-2-naphtalènecarbaldéhyde
o) trans-(5,6,7,8-tétrahydro-3,5,5,6,7,8,8-heptaméthyl-2-napthyl)-1-éthanone
ou tout mélange des composés m) et n) ou du composé o) avec le cis-(5,6,7,8-tétrahydro-3,5,5,6,7,8,8-heptaméthyl-2-naphtyl)-1-éthanone.

6. Utilisation d'un composé selon la revendication 1 à titre d'ingrédient parfumant.

7. Composition parfumante contenant à titre d'ingrédient actif un composé selon la revendication 1.

8. Produit parfumé contenant à titre d'ingrédient parfumant actif un composé selon la revendication 1.

9. A titre de produit parfumé selon la revendication 8, un parfum ou une eau de toilette, une lotion après-rasage, un savon, un gel de bain ou douche, un désodorisant corporel, une préparation cosmétique, un détergent ou un revitalisant textile.

10. Procédé de préparation d'un composé selon la revendication 1, caractérisé en ce qu'on
A.
a) fait réagir un composé de formule

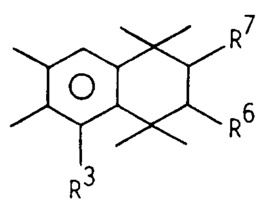

(IIIa)

dans laquelle les symboles $R^3$, $R^6$ et $R^7$, identiques ou différents, représentent chacun un atome d'hydrogène ou un radical méthyle,
avec un agent d'halogénation, sous l'action de la lumière, pour obtenir un mélange d'halogénures de formule

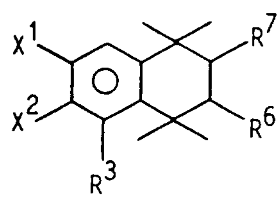

(IV)

dans laquelle les symboles $R^6$ et $R^7$ sont définis comme ci-dessus, le symbole $R^3$ représente un atome d'hydrogène, un atome d'halogène ($X^1 = X^2 = CH_3$) ou un radical méthyle et les

symboles $X^1$ et $X^2$ sont identiques et représentent chacun un radical méthyle ($R^3$ = halogène) ou sont différents et représentent chacun un atome d'halogène ou un radical méthyle ($R^3$ = H, $CH_3$);
b) on hydrolyse ensuite ledit mélange d'halogénures pour obtenir un mélange d'alcools correspondants que l'on oxyde par la suite pour obtenir un mélange d'aldéhydes de formule

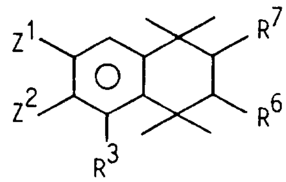

(Ia)

dans laquelle les symboles $R^6$ et $R^7$ sont définis comme ci-dessus, le symbole $R^3$ représente un atome d'hydrogène ou un radical méthyle, ou un groupe CHO lorsque $Z^1 = Z^2 = CH_3$, et les symboles $Z^1$ et $Z^2$ sont identiques et représentent chacun un radical méthyle lorsque $R^3$ = CHO ou sont différents et représentent chacun un groupe CHO ou un radical méthyle lorsque $R^3$ = H, $CH_3$;
c) on sépare lesdits aldéhydes et on les traite successivement avec MeLi ou MeMgX (Me = $CH_3$, X = halogène), $H_2O$ et un agent d'oxydation pour obtenir des cétones de formule

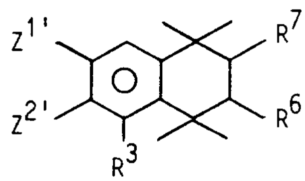

(Ib)

dans laquelle les symboles $R^6$ et $R^7$ ont le sens indiqué ci-dessus, le symbole $R^3$ représente un atome d'hydrogène ou un radical méthyle, ou un groupe $CH_3CO$ lorsque $Z^{1'} = Z^{2'} = CH_3$, et les symboles $Z^{1'}$ et $Z^{2'}$ sont identiques et représentent chacun un radical méthyle lorsque $R^3 = CH_3CO$ ou sont différents et représentent chacun un groupe $CH_3CO$ ou un radical méthyle lorsque $R^3$ = H, $CH_3$ ;

ou

B.

a) fait réagir un composé de formule

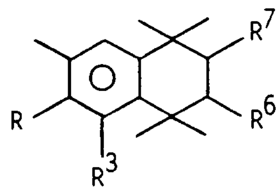

(IIIb)

dans laquelle les symboles R et $R^3$ sont différents et représentent chacun un atome d'hydrogène ou un radical méthyle et les symboles $R^6$ et $R^7$ sont définis ci-dessus,
avec un agent d'oxydation ou de formylation pour obtenir un aldéhyde de formule

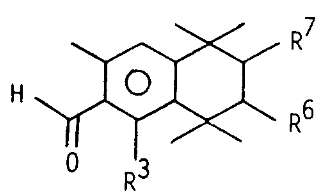

(Ic)

dans laquelle le symbole $R^3$ représente un atome d'hydrogène ou un radical méthyle ;
b) traite ensuite l'aldéhyde de formule (Ic) avec MeLi ou un réactif de Grignard, suivi d'un agent hydrolysant et d'un agent oxydant pour obtenir une cétone de formule

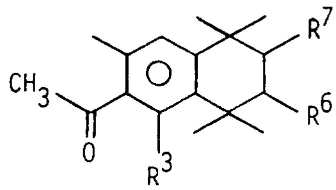

(Id)

dans laquelle les symboles $R^3$, $R^6$ et $R^7$ représentent chacun un atome d'hydrogène ou un radical méthyle ;
ou
C. fait réagir un composé de formule

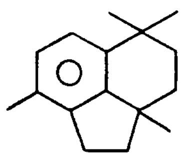

(IIIc)

ou un mélange de composés de formule

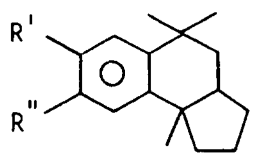

(IIId)

dans laquelle les symboles R' et R'' sont différents et représentent chacun un atome d'hydrogène ou un radical méthyle,
avec $Cl_2CHOCH_3$, dans les conditions de la réaction d'acylation de Friedel-Crafts, pour obtenir respectivement un aldéhyde de formule

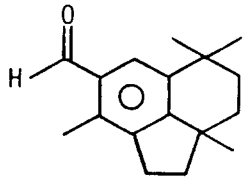

(Ie)

ou un mélange d'aldéhydes de formule

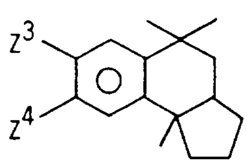

(If)

dans laquelle les symboles $Z^3$ et $Z^4$ sont différents et représentent chacun un groupe CHO ou un radical méthyle ;
ou avec chlorure d'acétyle, en présence d'un acide de Lewis, pour obtenir respectivement une

cétone de formule

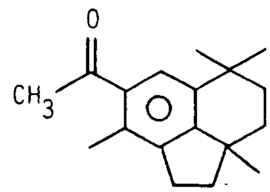

(Ig)

ou un mélange de cétones de formule

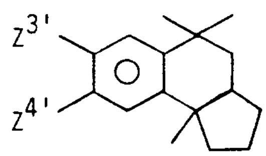

(Ih)

dans laquelle les symboles $Z^{3'}$ et $Z^{4'}$ sont différents et représentent chacun un groupe $CH_3CO$ ou un radical méthyle.

**11.** Composés de formule

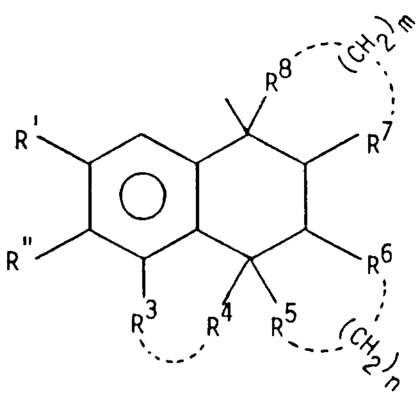

(III)

dans laquelle

a) les indices m et n définissent des nombres entiers identiques de valeur 0, les symboles $R^5$ et $R^8$ représentent chacun un radical méthyle, les symboles $R^6$ et $R^7$, identiques ou différents représentent chacun un atome d'hydrogène ou un radical méthyle, et soit le symbole $R^4$ représente un radical méthyle, les symboles R' et R" sont identiques et représentent chacun un radical méthyle et le symbole $R^3$ représente un atome d'hydrogène ou un radical méthyle, soit le symbole $R^4$ représente un radical méthyle, le symbole R" un atome d'hydrogène et les symboles R' et $R^3$ chacun un radical méthyle, soit les symboles $R^3$ et $R^4$ sont identiques et représentent chacun un radical méthylène faisant partie d'un cycle tel qu'indiqué par la ligne pointillée, et les symboles R' et R" représentent respectivement un atome d'hydrogène et un radical méthyle, la combinaison suivante étant cependant exclue :

1. $R^3 = R^4 = CH_2$ et $R^6$ ou $R^7 = CH_3$ ;

ou dans laquelle

b) les indices m et n sont différents et définissent chacun un nombre entier de valeur 0 ou 1, les symboles R' et R" représentent respectivement un atome d'hydrogène et un radical méthyle, le symbole $R^3$ représente un atome d'hydrogène, le symbole $R^4$ représente un radical méthyle, et soit les symboles $R^5$ et $R^6$ sont identiques (n = 1) et représentent chacun un groupe méthylène faisant partie d'un cycle tel qu'indiqué par la ligne pointillée, le symbole $R^7$ représentant un atome d'hydrogène et le symbole $R^8$ un radical méthyle, soit le symbole $R^5$ représente un radical méthyle et le symbole $R^6$ un atome d'hydrogène, les symboles $R^7$ et $R^8$ étant alors identiques (m = 1) et

représentant chacun un radical méthylène faisant partie d'un cycle tel qu'indiqué par la ligne pointillée,

ou tout mélange de deux ou plusieurs isomères structurels de formule (III).

**12.** A titre de composé selon la revendication 11, l'un des composés suivants :
1. trans-1,2,3,4-tétrahydro-1,1,2,3,4,4,6,7-octaméthylnaphtalène
2. cis-1,2,3,4-tétrahydro-1,1,2,3,4,4,6,7-octaméthylnaphtalène
ou tout mélange de ces deux isomères.

## Claims

**1.** A compound of formula

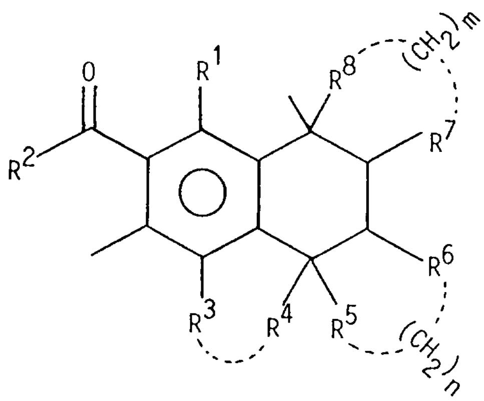

(I)

wherein

a) indexes m and n are identical and stand each for an integer number equal to zero, symbols $R^1$ and $R^2$ are identical and represent each a hydrogen atom, or are different and represent each a hydrogen atom or a methyl radical, symbols $R^5$ and $R^8$ stand each for a methyl radical, symbols $R^6$ and $R^7$ can be identical or different and designate each a hydrogen atom or a methyl radical and, either symbol $R^4$ represents a methyl radical and symbol $R^3$ stands for a hydrogen atom or a methyl radical, or symbols $R^3$ and $R^4$ represent each a methylene radical belonging to a ring such as indicated by the dotted line, with the proviso that the following combinations are excluded :

1. $R^1 = R^2 = R^3 = R^6 = R^7 = H$, or
2. $R^1 = R^2 = R^3 = H$ and $R^6$ or $R^7 = CH_3$, or
3. $R^2 = CH_3$ and $R^3 = R^6 = R^7 = H$, or
4. $R^2 = CH_3$, $R^1 = R^3 = H$ and $R^6$ or $R^7 = CH_3$, or
5. $R^1 = R^3 = CH_3$, or
6. $R^3 = R^4 = CH_2$ and $R^6$ or $R^7 = CH_3$ ;

or wherein

b) indexes m and n are different and define each an integer number equal to 0 or 1, symbol $R^2$ stands for a hydrogen atom or a methyl radical, symbols $R^1$ and $R^3$ designate each a hydrogen atom, symbol $R^4$ represents a methyl radical and, either symbols $R^5$ and $R^6$ are identical (n = 1) and represent each a methylene radical belonging to a ring such as indicated by the dotted line, symbol $R^7$ representing a hydrogen atom and symbol $R^8$ a methyl radical, or symbol $R^5$ stands for a methyl radical and symbol $R^6$ for a hydrogen atom, symbols $R^7$ and $R^8$ being then identical (m = 1) and designating each a methylene radical belonging to a ring such as indicated by the dotted line;

or any mixture of two or more structural isomers of formula (I).

**2.** As a compound of formula (I) according to claim 1, a compound of formula

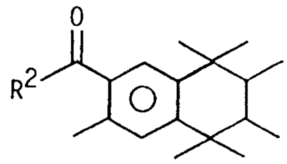

(II)

wherein symbol $R^2$ stands for a hydrogen atom or a methyl radical.

**3.** As a compound of formula (I) according to claim 1, one of the following compounds:
a) 5,6,7,8-tetrahydro-1,3,5,5,8,8-hexamethyl-2-naphthalenecarbaldehyde
b) 5,6,7,8-tetrahydro-3,4,5,5,8,8-hexamethyl-2-naphthalenecarbaldehyde
c) 5,6,7,8-tetrahydro-1,3,5,5,6,8,8-heptamethyl-2-naphthalenecarbaldehyde
d) 5,6,7,8-tetrahydro-3,4,5,5,7,8,8-heptamethyl-2-naphthalenecarbaldehyde
e) 5,6,7,8-tetrahydro-1,3,5,5,7,8,8-heptamethyl-2-naphthalenecarbaldehyde
f) 5,6,7,8-tetrahydro-3,4,5,5,6,8,8-heptamethyl-2-naphthalenecarbaldehyde
g) 1,2,6,7,8,8a-hexahydro-3,6,6,8a-tetramethyl-4-acenaphthylenecarbaldehyde
h) (1,2,6,7,8,8a-hexahydro-3,6,6,8a-tetramethyl-4-acenaphthylenyl)-1-ethanone
or any mixture of compounds a) and b), or c) and d), or e) and f).

**4.** As a compound according to claim 1, a mixture of following compounds i) and j) or k) and l):
i) 2,3,3a,4,5,9b-hexahydro-5,5,8,9b-tetramethyl-1H-benz[e]inden-7-carbaldehyde
j) 2,3,3a,4,5,9b-hexahydro-5,5,7,9b-tetramethyl-1H-benz[e]inden-8-carbaldehyde
k) 1-(2,3,3a,4,5,9b-hexahydro-5,5,8,9b-tetramethyl-1H-benz[e]inden-7-yl)-1-ethanone
l) 1-(2,3,3a,4,5,9b-hexahydro-5,5,7,9b-tetramethyl-1H-benz[e]inden-8-yl)-1-ethanone.

**5.** As a compound according to claim 2, one of the following compounds :
m) trans-5,6,7,8-tetrahydro-3,5,5,6,7,8,8-heptamethyl-2-naphthalenecarbaldehyde
n) cis-5,6,7,8-tetrahydro-3,5,5,6,7,8,8-heptamethyl-2-naphthalenecarbaldehyde
o) trans-(5,6,7,8-tetrahydro-3,5,5,6,7,8,8-heptamethyl-2-naphthyl)-1-ethanone
or any mixture of compounds m) and n) or of compound o) with cis-(5,6,7,8-tetrahydro-3,5,5,6,7,8,8-heptamethyl-2-naphthyl)-1-ethanone.

**6.** Use of a compound according to claim 1 as a perfuming ingredient.

**7.** A perfuming composition containing as an active ingredient a compound according to claim 1.

**8.** A perfumed article containing as an active perfuming ingredient a compound according to claim 1.

**9.** As a perfumed article according to claim 8, a perfume or Cologne, an after-shave lotion, a soap, a bath or shower gel, a body deodorant, a cosmetic preparation, a detergent or fabric softener.

**10.** A process for the preparation of a compound according to claim 1, characterized in that :
A.
a) there is reacted, under the action of light, a compound of formula

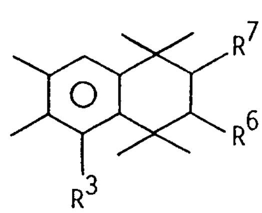

(IIIa)

wherein symbols $R^3$, $R^6$ and $R^7$ can be identical or different and represent each a hydrogen

atom or a methyl radical,
with a halogenation agent to obtain a mixture of halides of formula

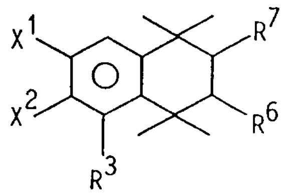

(IV)

wherein symbols $R^6$ and $R^7$ are defined as above, symbol $R^3$ stands for a hydrogen atom, a halogen atom ($X^1 = X^2 = CH_3$) or a methyl radical, and symbols $X^1$ and $X^2$ are identical and designate each a methyl radical ($R^3$ = halogen) or are different ($R^3 = H$, $CH_3$) and represent each a halogen atom or a methyl radical ;

b) said mixture of halides is then hydrolysed to obtain a mixture of corresponding alcohols, which is subsequently oxidized to provide a mixture of aldehydes of formula

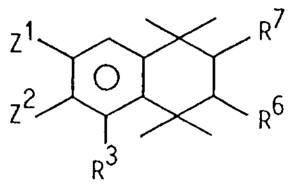

(Ia)

wherein symbols $R^6$ and $R^7$ are defined as above, symbol $R^3$ represents a hydrogen atom or a methyl radical, or a CHO group when $Z^1 = Z^2 = CH_3$, and symbols $Z^1$ and $Z^2$ are identical and designate each a methyl radical when $R^3$ = CHO, or are different and represent each a CHO group or a methyl radical when $R^3 = H$, $CH_3$ ;

c) said aldehydes are separated from the reaction mixture, and treated successively with MeLi or MeMgX (Me = $CH_3$, X = halogen), $H_2O$ and an oxidation agent, to obtain ketones of formula

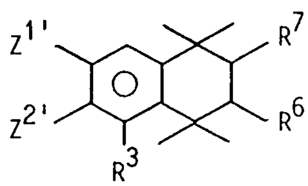

(Ib)

wherein symbols $R^6$ and $R^7$ have the meaning indicated above, symbol $R^3$ represents a hydrogen atom or a methyl radical, or a $CH_3CO$ group when $Z^{1'} = Z^{2'} = CH_3$, and symbols $Z^{1'}$ and $Z^{2'}$ are identical and designate each a methyl radical when $R^3 = CH_3CO$ or are different and represent each a $CH_3CO$ group or a methyl radical when $R^3 = H$, $CH_3$ ;

or

B.

a) a compound of formula

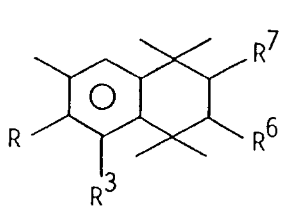

(IIIb)

wherein symbols R and $R^3$ are different and represent each a hydrogen atom or a methyl radical and symbols $R^6$ and $R^7$ are defined as above,
is reacted with an oxidation or formylation agent to obtain an aldehyde of formula

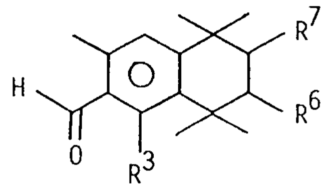

(Ic)

wherein symbol $R^3$ represents a hydrogen atom or a methyl radical ;
and
b) the aldehyde of formula (Ic) is then treated with MeLi or a Grignard reagent followed by a hydrolysing agent and an oxidizing agent to obtain a ketone of formula

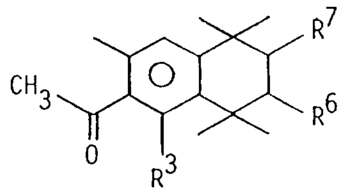

(Id)

wherein symbols $R^3$, $R^6$ and $R^7$ designate each a hydrogen atom or a methyl radical ;
or
C. a compound of formula

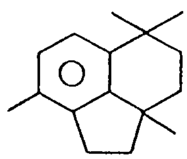

(IIIc)

or a mixture of compounds of formula

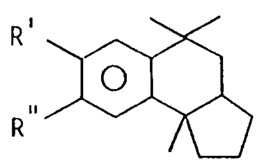

(IIId)

wherein symbols R' and R'' are different and represent each a hydrogen atom or a methyl radical,
is reacted with $Cl_2CHOCH_3$, under the Friedel-Crafts acylation reaction conditions, to obtain respectively an aldehyde of formula

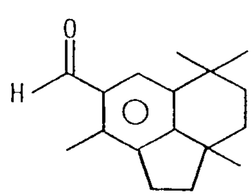

(Ie)

or a mixture of aldehydes of formula

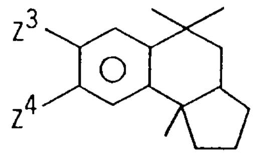

(If)

wherein symbols $Z^3$ and $Z^4$ are different and represent each a CHO group or a methyl radical ; or with acetyl chloride, in the presence of a Lewis acid, to obtain respectively a ketone of formula

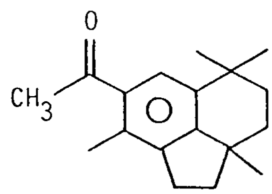

(Ig)

or a mixture of ketones of formula

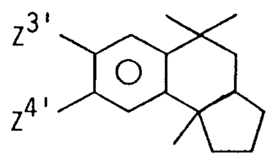

(Ih)

wherein symbols $Z^{3'}$ and $Z^{4'}$ are different and designate each a $CH_3CO$ group or a methyl radical.

**11.** Compounds of formula

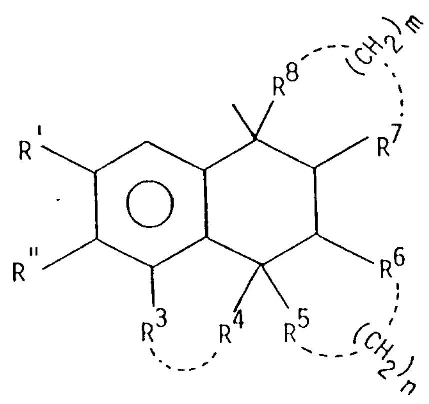

(III)

wherein

a) indexes m and n define identical integer numbers equal to zero, symbols $R^5$ and $R^8$ represent each a methyl radical, symbols $R^6$ and $R^7$ can be identical or different and represent each a hydrogen atom or a methyl radical, and either symbol $R^4$ represents a methyl radical, symbols R' and R'' are identical and stand each for a methyl radical and symbol $R^3$ represents a hydrogen atom or a methyl radical, or symbol $R^4$ designates a methyl radical, symbol R'' a hydrogen atom and symbols R' and $R^3$ each a methyl radical, or symbols $R^3$ and $R^4$ are identical and designate each a methylene radical belonging to a ring such as indicated by the dotted line and symbols R' and R'' designate respectively a hydrogen atom and a methyl radical, with the proviso that the following combination is excluded :

1. $R^3 = R^4 = CH_2$ and $R^6$ or $R^7 = CH_3$;

or wherein

b) indexes m and n are different and define each an integer number equal to 0 or 1, symbols R' and R'' represent respectively a hydrogen atom and a methyl radical, symbol $R^3$ stands for a hydrogen atom, symbol $R^4$ represents a methyl radical and, either symbols $R^5$ and $R^6$ are identical (n = 1) and stand each for a methylene group belonging to a ring such as represented by the dotted line, symbol $R^7$ designating a hydrogen atom and symbol $R^8$ a methyl radical, or symbol $R^5$ designates a methyl radical and symbol $R^6$ a hydrogen atom, symbols $R^7$ and $R^8$ being then identical (m = 1) and standing each for a methylene radical belonging to a cycle such as indicated by the dotted line,

or any mixture of two or more structural isomers of formula (III).

**12.** As a compound according to claim 11, one of the following compounds :

1. trans-1,2,3,4-tetrahydro-1,1,2,3,4,4,6,7-octamethylnaphthalene
2. cis-1,2,3,4-tetrahydro-1,1,2,3,4,4,6,7-octamethylnaphthalene

or any mixture of these two isomers.

**Patentansprüche**

**1.** Verbindung der Formel

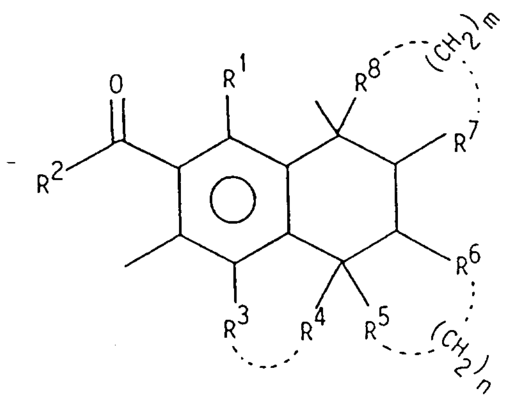

(I)

worin

a) die Indices m und n gleich sind und jeweils eine ganze Zahl vom Wert 0 definieren, die Symbole $R^1$ und $R^2$ gleich sind und jeweils ein Wasserstoffatom darstellen oder verschieden sind und jeweils ein Wasserstoffatom oder einen Methylrest bedeuten, die Symbole $R^5$ und $R^8$ jeweils einen Methylrest bedeuten, die Symbole $R^6$ und $R^7$, gleich oder verschieden, jeweils ein Wasserstoffatom oder einen Methylrest bedeuten und entweder das Symbol $R^4$ für einen Methylrest und das Symbol $R^3$ für ein Wasserstoffatom oder einen Methylrest steht oder die Symbole $R^3$ und $R^4$ jeweils einen Methylenrest bedeuten, der Bestandteil eines Ringes ist, wie dies durch die punktierte Linie angegeben wird, wobei jedoch die nachfolgenden Kombinationen ausgeschlossen sind

1. $R^1 = R^2 = R^3 = R^6 = R^7 = H$ oder
2. $R^1 = R^2 = R^3 = H$ und $R^6$ oder $R^7 = CH_3$ oder
3. $R^2 = CH_3$ und $R^3 = R^6 = R^7 = H$ oder
4. $R^2 = CH_3$, $R^1 = R^3 = H$ und $R^6$ oder $R^7 = CH_3$ oder
5. $R^1 = R^3 = CH_3$ oder
6. $R^3 = R^4 = CH_2$ und $R^6$ oder $R^7 = CH_3$;

oder worin

b) die Indices m und n verschieden sind und jeweils eine ganze Zahl vom Wert 0 oder 1 definieren, das Symbol $R^2$ für ein Wasserstoffatom oder einen Methylrest steht, jedes der Symbole $R^1$ und $R^3$ ein Wasserstoffatom bedeutet, das Symbol $R^4$ einen Methylrest bedeutet und entweder die Symbole $R^5$ und $R^6$ gleich sind (n = 1) und jeweils für einen Methylenrest stehen, der Bestandteil eines Ringes ist, wie dies durch die punktierte Linie angegeben wird, das Symbol $R^7$ für ein Wasserstoffatom und das Symbol $R^8$ für einen Methylrest stehen, oder das Symbol $R^5$ einen Methylrest und das Symbol $R^6$ ein Wasserstoffatom bedeuten, die Symbole $R^7$ und $R^8$ dann gleich sind (m = 1) und jeweils für einen Methylenrest stehen, der Bestandteil eines Ringes ist, wie dies durch die punktierte

Linie angegeben wird;
oder jede Mischung von zwei oder mehreren Strukturisomeren der Formel (I).

**2.** Als Verbindung der Formel (I) gemäss Patentanspruch 1 eine Verbindung der Formel

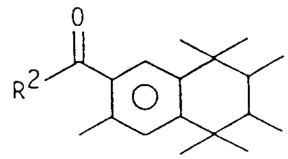

(II)

worin das Symbol $R^2$ für ein Wasserstoffatom oder einen Methylrest steht.

**3.** Als Verbindung der Formel (I) gemäss Patentanspruch 1 eine der nachfolgenden Verbindungen:
a) 5,6,7,8-Tetrahydro-1,3,5,5,8,8-hexamethyl-2-naphthalincarbaldehyd
b) 5,6,7,8-Tetrahydro-3,4,5,5,8,8-hexamethyl-2-naphthalincarbaldehyd
c) 5,6,7,8-Tetrahydro-1,3,5,5,6,8,8-heptamethyl-2-naphthalincarbaldehyd
d) 5,6,7,8-Tetrahydro-3,4,5,5,7,8,8-heptamethy-2-naphthalincarbaldehyd
e) 5,6,7,8-Tetrahydro-1,3,5,5,7,8,8-heptamethyl-2-naphthalincarbaldehyd
f) 5,6,7,8-Tetrahydro-3,4,5,5,6,8,8-heptamethyl-2-naphthalincarbaldehyd
g) 1,2,6,7,8,8a-Hexahydro-3,6,6,8a-tetramethyl-4-acenaphthylencarbaldehyd
h) (1,2,6,7,8,8a-Hexahydro-3,6,6,8a-tetramethyl-4-acenaphthylenyl)1-ethanon
oder jede Mischung der Verbindungen a) und b) oder c) und d) oder e) und f).

**4.** Als Verbindung gemäss Patentanspruch 1 ein Gemisch der nachfolgenden Verbindungen i) und j) oder k) und l):
i) 2,3,3a,4,5,9b-Hexahydro-5,5,8,9b-tetramethyl-1H-benz[e]inden-7-carbaldehyd
j) 2,3,3a,4,5,9b-Hexahydro-5,5,7,9b-tetramethyl-1H-benz[e]inden-8-carbaldehyd
k) 1-(2,3,3a,4,5,9b-Hexahydro-5,5,8,9b-tetramethyl-1H-benz[e]inden-7-yl)-1-ethanon
l) 1-(2,3,3a,4,5,9b-Hexahydro-5,5,7,9b-tetramethyl-1H-benz[e]inden-8-yl)-1-ethanon.

**5.** Als Verbindung gemäss Patentanspruch 2 eine der nachfolgenden Verbindungen:
m) trans-5,6,7,8-Tetrahydro-3,5,5,6,7,8,8-heptamethyl-2-naphthalincarbaldehyd
n) cis-5,6,7,8-Tetrahydro-3,5,5,6,7,8,8-heptamethyl-2-naphthalincarbaldehyd
o) trans-(5,6,7,8-Tetrahydro-3,5,5,6,7,8,8-heptamethyl-2-naphthyl)-1-ethanon
oder jede Mischung der Verbindungen m) und n) oder der Verbindung o) mit cis-(5,6,7,8-Tetrahydro-3,5,5,6,7,8,8-heptamethyl-2-naphthyl)-1-ethanon.

**6.** Verwendung einer Verbindung gemäss Patentanspruch 1 als Riechstoffbestandteil.

**7.** Riechstoffzusammensetzung, enthaltend als aktiven Bestandteil eine Verbindung gemäss Patentanspruch 1.

**8.** Parfümiertes Produkt, enthaltend als aktiven Riechstoffbestandteil eine Verbindung gemäss Patentanspruch 1.

**9.** Als parfümiertes Produkt gemäss Patentanspruch 8 ein Parfüm oder ein Toilettwasser, eine Rasierlotion, eine Seife, ein Bade-oder Duschgel, ein Körperdesodorant, eine kosmetische Zusammensetzung, ein Reinigungsmittel oder ein Textilauffrischungsmittel.

**10.** Verfahren zur Herstellung einer Verbindung gemäss Patentanspruch 1, dadurch gekennzeichnet, dass man

A.

a) eine Verbindung der Formel

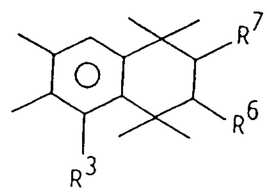

(IIIa)

worin die Symbole $R^3$, $R^6$ und $R^7$, gleich oder verschieden, jeweils für ein Wasserstoffatom oder einen Methylrest stehen,
mit einem Halogenierungsmittel unter Einwirkung von Licht umsetzt, um ein Gemisch von Halogeniden der Formel

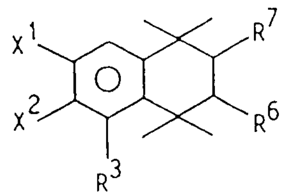

(IV)

zu erhalten, worin die Symbole $R^6$ unf $R^7$ die obige Bedeutung besitzen, das Symbol $R^3$ ein Wasserstoffatom, ein Halogenatom ($X^1 = X^2 = CH_3$) oder einen Methylrest bedeutet und die Symbole $X^1$ und $X^2$ gleich sind und jeweils für einen Methylrest ($R^3$ = Halogen) stehen oder verschieden sind und jeweils für ein Halogenatom oder einen Methylrest ($R^3 = H, CH_3$) stehen;

b) anschliessend dieses Halogenidgemisch hydrolysiert, um ein Gemisch der entsprechenden Alkohole zu erhalten, welche man anschliessend oxidiert, um ein Gemisch von Aldehyden der Formel

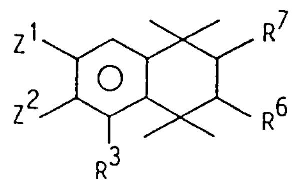

(Ia)

zu erhalten, worin die Symbole $R^6$ und $R^7$ die obige Bedeutung besitzen, das Symbol $R^3$ für ein Wasserstoffatom oder einen Methylrest steht oder eine CHO-Gruppe bedeutet, wenn $Z^1 = Z^2 = CH_3$, und die Symbole $Z^1$ und $Z^2$ gleich sind und jeweils für einen Methylrest stehen, wenn $R^3$ = CHO, oder verschieden sind und jeweils eine CHO-Gruppe oder einen Methylrest bedeuten, wenn $R^3 = H, CH_3$,

c) diese Aldehyde trennt und sie nacheinander mit MeLi oder MeMgX (Me = $CH_3$, X = Halogen), $H_2O$ und einem Oxidationsmittel behandelt, um Ketone der Formel

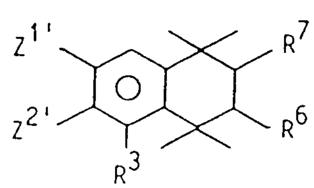

(Ib)

zu erhalten, worin die Symbole $R^6$ und $R^7$ die oben angegebene Bedeutung besitzen, das

Symbol $R^3$ für ein Wasserstoffatom oder einen Methylrest steht oder eine $CH_3CO$-Gruppe bedeutet, wenn $Z^{1'} = Z^{2'} = CH_3$, und die Symbole $Z^{1'}$ und $Z^{2'}$ gleich sind und jeweils für einen Methylrest stehen, wenn $R^3 = CH_3CO$, oder verschieden sind und jeweils für eine $CH_3CO$-Gruppe oder für einen Methylrest stehen, wenn $R^3 = H, CH_3$,

oder

B.

a) eine Verbindung der Formel

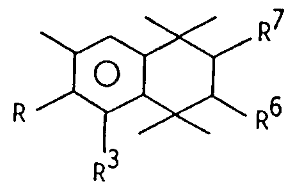

(IIIb)

worin die Symbole R und $R^3$ verschieden sind und jeweils ein Wasserstoffatom oder einen Methylrest bedeuten und die Symbole $R^6$ und $R^7$ die obige Bedeutung besitzen,
mit einem Oxidations- oder Formylierungsmittel umsetzt, um einen Aldehyd der Formel

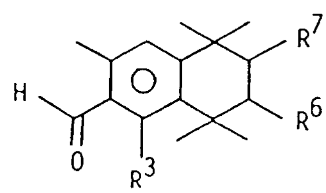

(Ic)

zu erhalten, worin das Symbol $R^3$ für ein Wasserstoffatom oder einen Methylrest steht;

b) anschliessend den Aldehyd der Formel (Ic) mit MeLi oder einem Grignard-Reagenz behandelt, gefolgt von einem Hydrolysierungsmittel und einem Oxidationsmittel, um ein Keton der Formel

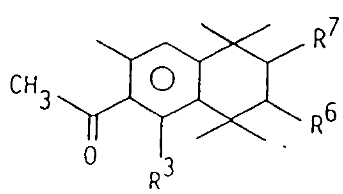

(Id)

zu erhalten, worin die Symbole $R^3$, $R^6$ und $R^7$ jeweils für ein Wasserstoffatom oder einen Methylrest stehen;

oder

C. eine Verbindung der Formel

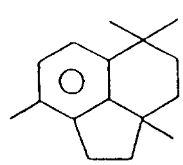

(IIIc)

oder ein Gemisch von Verbindungen der Formel

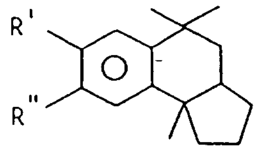

(IIId)

worin die Symbole R' und R'' verschieden sind und jeweils ein Wasserstoffatom oder einen Methylrest bedeuten, mit $Cl_2CHOCH_3$ unter den Bedingungen einer Friedl-Crafts-Acylierung umsetzt, um einen Aldehyd der Formel

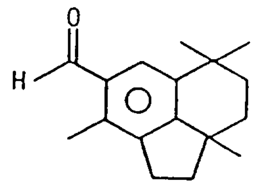

(Ie)

oder ein Gemisch von Aldehyden der Formel

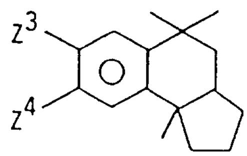

(If)

zu erhalten, worin die Symbole $Z^3$ und $Z^4$ verschieden sind und jeweils eine CHO-Gruppe oder einen Methylrest bedeuten; oder mit Acetylchlorid in Gegenwart einer Lewis-Säure umsetzt, um ein Keton der Formel

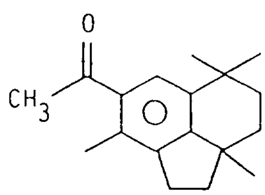

(Ig)

oder ein Gemisch von Ketonen der Formel

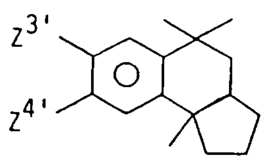

(Ih)

zu erhalten, worin die Symbole $Z^{3'}$ und $Z^{4'}$ verschieden sind und jeweils eine $CH_3CO$-Gruppe oder einen Methylrest bedeuten.

**11.** Verbindungen der Formel

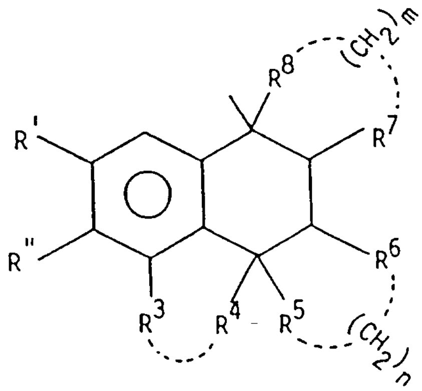

(III)

worin

a) die Indices m und n gleiche ganze Zahlen vom Wert 0 definieren, jeweils der Symbole $R^5$ und $R^8$ einen Methylrest bedeutet, die Symbole $R^6$ und $R^7$, gleich oder verschieden, jeweils für ein Wasserstoffatom oder einen Methylrest stehen, und entweder das Symbol $R^4$ einen Methylrest bedeutet, die Symbole R' und R'' gleich sind und jeweils einen Methylrest bedeuten und das Symbol $R^3$ für ein Wasserstoffatom oder einen Methylrest steht, oder das Symbol $R^4$ einen Methylrest bedeutet, das Symbol R'' ein Wasserstoffatom und jeweils der Symbole R' und $R^3$ einen Methylrest bedeutet, oder die Symbole $R^3$ oder $R^4$ gleich sind und jeweils für einen Methylenrest stehen, der Bestandteil eines Ringes ist, wie dies durch die punktierte Linie angegeben wird, und die Symbole R' und R'' beziehungsweise für ein Wasserstoffatom und einen Methylrest stehen, wobei die nachfolgende Kombination jedoch ausgeschlossen ist : 1. $R^3 = R^4 = CH_2$ und $R^6$ oder $R^7 = CH_3$;

oder worin

b) die Indices m und n verschieden sind und jeweils eine ganze Zahl vom Wert 0 oder 1 definieren, die Symbole R' und R'' beziehungsweise für ein Wasserstoffatom und einen Methylrest stehen, das Symbol $R^3$ ein Wasserstoffatom bedeutet, das Symbol $R^4$ für einen Methylrest steht, und entweder die Symbole $R^5$ und $R^6$ gleich sind (n = 1) und jeweils für einen Methylenrest stehen, der Bestandteil eines Ringes ist, wie dies durch die punktierte Linie angegeben wird, das Symbol $R^7$ ein Wasserstoffatom und das Symbol $R^8$ einen Methylrest bedeuten, oder das Symbol $R^5$ für einen Methylrest und das Symbol $R^6$ für ein Wasserstoffatom stehen, die Symbole $R^7$ und $R^8$ dann gleich sind (m = 1) und jeweils für einen Methylenrest stehen, der Bestandteil eines Ringes ist, wie dies durch die punktierte Linie angegeben wird,

oder jede Mischung von zwei oder mehreren Strukturisomeren der Formel (III).

**12.** Als Verbindung gemäss Patentanspruch 11 eine der nachfolgenden Verbindungen:
1. trans-1,2,3,4-Tetrahydro-1,1,2,3,4,4,6,7-oktamethylnaphthalin
2. cis-1,2,3,4-Tetrahydro-1,1,2,3,4,4,6,7-oktamethylnaphthalin oder jede Mischung dieser beiden Isomeren.